(19) Europäisches Patentamt
European Patent Office
Office européen des brevets

(11) Veröffentlichungsnummer: **0 358 606**
**A2**

(12) # EUROPÄISCHE PATENTANMELDUNG

(21) Anmeldenummer: **89810647.1**

(22) Anmeldetag: **01.09.89**

(51) Int. Cl.5: **C 07 H 19/01**
C 12 P 19/02, C 12 N 1/21,
A 01 N 43/90
//(C12P19/02,C12R1:01),
(C12N1/21,C12R1:01)

(30) Priorität: **09.09.88 CH 3378/88**
**12.09.88 CH 3395/88**

(43) Veröffentlichungstag der Anmeldung:
**14.03.90 Patentblatt 90/11**

(84) Benannte Vertragsstaaten:
**AT BE CH DE ES FR GB GR IT LI LU NL SE**

(71) Anmelder: **Gesellschaft für Biotechnologische Forschung mbH (GBF)**
**Mascheroder Weg 1**
**D-3300 Braunschweig-Stöckheim (DE)**

**CIBA-GEIGY AG**
**Klybeckstrasse 141**
**CH-4002 Basel (CH)**

(72) Erfinder: **Bedorf, Norbert, Dr.**
**Krukenbergstrasse 4**
**D-3308 Königslutter (DE)**

**Böhlendorf, Bettina**
**Zimmerstrasse 2**
**D-3300 Braunschweig (DE)**

**Forche, Edgar, Dr.**
**Schlosserweg 2**
**D-3300 Braunschweig (DE)**

**Gerth, Klaus, Dr.**
**Am Butterbusch 17**
**D-3300 Braunschweig (DE)**

**Höfle, Gerhard, Prof. Dr.**
**Alter Weg 12a**
**D-3300 Braunschweig (DE)**

**Irschik, Herbert, Dr.**
**Masurenweg 15**
**D-3340 Wolfenbüttel (DE)**

**Jansen, Rolf, Dr.**
**Falkenbergstrasse 14**
**D-3300 Braunschweig (DE)**

**Kunze, Brigitte, Dr.**
**Hinter Aegidien 5**
**D-3300 Braunschweig (DE)**

**Reichenbach, Hans, Prof. Dr.**
**Platanenstrasse 35**
**D-3340 Wolfenbüttel (DE)**

**Sasse, Florenz, Dr.**
**Im Rübenkamp 7**
**D-3300 Braunschweig (DE)**

**Steinmetz, Heinrich**
**Magdalenenweg 15**
**D-3320 Hildesheim-Sorsum (DE)**

**Trowitzsch-Kienast, Wolfram, Dr.**
**Am Hasengarten 8**
**D-3300 Braunschweig (DE)**

**Pachlatko, Johannes Paul, Dr.**
**Bölchenstrasse 21**
**CH-4411 Seltisberg (CH)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 5397, DSM 4795, DSM 4796, DSM 4797, DSM 5393 und DSM 5414.
Die Bezeichnung der Erfindung wurde geändert (Richtlinien für die Prüfung im EPA, A-III, 7.3).

EP 0 358 606 A2

(54) **Mikrobiologisches Verfahren zur Herstellung agrarchemisch verwendbarer mikrobizider makrozyklischer Lactonderivate.**

(57) Es wird ein mikrobiologisches Verfahren zur Herstellung von Verbindungen der Formel I

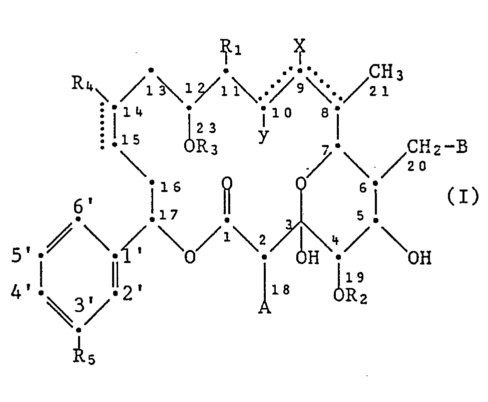

(I)

beschrieben, die sich in der Agrarchemie zur Bekämpfung bzw. Verhütung von Pflanzenkrankheiten verwenden lassen. Die Substituenten haben die in der Beschreibung gegebene Bedeutung. Es werden sechs neue Mikroorganismen zur Durchführung des Verfahrens beschrieben.

Trennungsgang zur Isolierung von Soraphenen

XAD-Eluat
Chromatographie 1-17
Soraphen A .. O
FIGUR 1

**Beschreibung**

## Mikrobiologisches Verfahren zur Herstellung agrarchemisch verwendbarer Wirkstoffe

Die vorliegende Erfindung betrifft ein mikrobiologisches Verfahren zur Herstellung von agrarchemisch verwendbaren macrocyclischen Wirkstoffen der Formel I, neue Mikroorganismen zur Durchführung des Verfahrens sowie die nach dem Verfahren gewonnenen Wirkstoffe bzw. das nach dem Verfahren gewonnene Substrat, das solche Wirkstoffe enthält, und die Verwendung der Wirkstoffe zur Bekämpfung und Verhütung von Pflanzenkrankheiten.

(I)

Die vorstehende Formel betrifft folgende Substituentenkombinationen, wobei der macrocyclische Ring entweder gesättigt ist oder wahlweise in der 8,9-Stellung oder in der 9,10-Stellung oder in der 14,15-Stellung eine Doppelbindung enthält:

| Verb. | $R_1$ | $R_2$ | $R_3$ | $R_4$ | $R_5$ | A | B | X | Y | Doppelbindung |
|---|---|---|---|---|---|---|---|---|---|---|
| A | $OCH_3$ | $CH_3$ | $CH_3$ | H | H | $CH_3$ | H | H | H | $\Delta$9,10 |
| B | OH | $CH_3$ | $CH_3$ | H | H | $CH_3$ | H | H | H | -- |
| C | OH | $CH_3$ | $CH_3$ | H | H | $CH_3$ | H | H | H | $\Delta$9,10 |
| D | OH | $CH_3$ | $CH_3$ | H | H | H | H | H | H | -- |
| E | $OCH_3$ | $CH_3$ | $CH_3$ | H | H | $CH_3$ | H | OH | H | -- |
| F | $OCH_3$ | $CH_3$ | $CH_3$ | H | H | $CH_3$ | H | H | H | -- |
| H | $OCH_3$ | $CH_3$ | H | H | H | $CH_3$ | H | H | OH | $\Delta$8,9 |
| J | $OCH_3$ | H | $CH_3$ | H | H | $CH_3$ | H | H | H | -- |
| M | OH | $CH_3$ | $CH_3$ | H | H | $CH_3$ | H | OH | H | -- |
| N | OH | $CH_3$ | $CH_3$ | H | H | $CH_3$ | H | H | $OCH_3$ | $\Delta$9,10 |
| Q | $OCH_3$ | $CH_3$ | $CH_3$ | H | H | H | H | H | H | -- |
| R | OH | $CH_3$ | H | H | H | H | H | H | H | -- |
| S | OH | $CH_3$ | H | H | H | $CH_3$ | H | H | H | $\Delta$9,10 |
| T | OH | $CH_3$ | H | H | H | $CH_3$ | H | H | H | -- |
| U | $OCH_3$ | $CH_3$ | $CH_3$ | OH | H | $CH_3$ | H | H | H | $\Delta$9,10 |
| V | $OCH_3$ | $CH_3$ | H | H | H | $CH_3$ | H | H | H | $\Delta$9,10 |
| X | OH | $CH_3$ | H | H | OH | $CH_3$ | H | H | H | $\Delta$9,10 |
| Y | =O | $CH_3$ | H | H | H | $CH_3$ | H | OH | H | -- |
| Z | $OCH_3$ | $CH_3$ | H | OH | H | $CH_3$ | H | H | H | $\Delta$9,10 |
| β | $OCH_3$ | $CH_3$ | $CH_3$ | H | H | $CH_3$ | H | H | H | $\Delta$9,10 |
| γ | OH | $CH_3$ | $CH_3$ | H | H | $CH_3$ | H | 9,10-epoxy | | -- |
| δ | OH | $CH_3$ | $CH_3$ | H | H | $CH_3$ | H | H | H | $\Delta$9,10 |
| ζ | OH | $CH_3$ | $CH_3$ | H | H | $CH_3$ | OH | H | H | $\Delta$9,10 |
| η | OH | $CH_3$ | $CH_3$ | OH | H | $CH_3$ | H | H | H | $\Delta$9,10 |
| κ | OH | $CH_3$ | H | H | H | $CH_3$ | H | H | H | $\Delta$9,10 |
| μ | $OCH_3$ | $CH_3$ | H | H | H | $CH_3$ | H | H | OH | -- |
| ν | OH | $CH_3$ | $CH_3$ | H | H | $CH_3$ | H | OH | H | $\Delta$14,15 |
| ε | OH | $CH_3$ | H | H | H | $CH_3$ | H | H | H | $\Delta$9,10 |
| o | $OCH_3$ | $CH_3$ | $CH_3$ | OH | H | $CH_3$ | H | H | H | $\Delta$9,10 |
| π | OH | $CH_3$ | H | H | H | $CH_3$ | H | H | H | $\Delta$9,10 |
| ρ | OH | $CH_3$ | H | H | H | $CH_3$ | H | OH | H | -- |
| σ | $OCH_3$ | $CH_3$ | $CH_3$ | OH | H | $CH_3$ | H | H | H | $\Delta$9,10 |

Diese 32 Verbindungen werden durch mikrobiologische Kultivierung von Myxobakterien aus der Sorangium/Polyangium-Gruppe gewonnen, von denen sie, in Abhängigkeit vom jeweiligen Stamm, sämtlich oder teilweise und in unterschiedlichen Verhältniszahlen produziert werden. Gemeinsam ist allen Stämmen die Produktion der Verbindung A als eines der Hauptprodukte. Die Verbindungen der Formel I sollen hier und im folgenden "Soraphene" genannt werden, mithin die Verbindungen der vorstehenden Aufzählung "Soraphen A" bis "Soraphen σ" genannt werden.

Die Erfindung betrifft insbesondere 6 neue Mikroorganismen-Stämme der cellulosezersetzenden Myxobakterien aus der Sorangium/Polyangium-Gruppe, die sich, wie bekannt, häufig in Erdproben, Pflanzenmaterial oder in Tier-Dung finden lassen. Charakteristisch für diese taxonomisch nicht allgemein verbindlich gegliederte Gruppe ist ihre Fähigkeit, auf Cellulose oder Cellulose-Abbauprodukten als einziger Kohlenstoffquelle zu gedeihen. Die 6 Stämme gemäss vorliegender Erfindung, gleichgültig, ob sie strenggenommen zur Sorangium/Polyangium-Gruppe oder nur einem taxonomisch verwandten Bereich angehören, sind gegenüber den weitaus meisten Vertretern dieser Gruppe weiterhin dadurch gekennzeichnet, dass sie mindestens eines der vorstehend genannten "Soraphene" der Formel I produzieren, und bevorzugt mindestens zwei "Soraphene", darunter "Soraphen A".

Die 6 Stämme tragen hier und im folgenden den Sammelnamen Sorangium-(Polyangium)cellulosum. Sie entstammen Erdproben, die zu unterschiedlichen Zeiten an verschiedenen Stellen Europas, Afrikas bzw. der USA gesammelt wurden. Sie wurden bei der Deutschen Sammlung von Mikroorganismen (DSM) in Braunschweig, Bundesrepublik Deutschland, gemäss Budapester Vertrag hinterlegt. Sie sind wie folgt gekennzeichnet.

1.) Sorangium(Polyangium)cellulosum Stamm "So ce 139", isoliert im Mai 1986 aus einer im Herbst 1982 bei Fort Huachaca, Arizona, USA, gesammelten Erdprobe. Hinterlegungsnummer: DSM 5397. Hinterlegungstag: 2 Juni 1989.

2.) Sorangium(Polyangium)cellulosum Stamm "So ce 170", isoliert im April 1987 aus einer im Mai 1981 gesammelten Erdprobe von der Insel Delos, Griechenland. Hinterlegungsnummer: DSM 4795.

Hinterlegungstag: 2 September 1988.

3.) Sorangium(Polyangium)cellulosum Stamm "So ce 191", isoliert im August 1987 aus einer im Februar 1987 gesammelten Erdprobe von der Insel Madeira, Portugal. Hinterlegungsnummer: DSM 4796. Hinterlegungstag: 2. September 1988.

4.) Sorangium(Polyangium)cellulosum "So ce 192", isoliert im August 1987 aus einer im März 1987 gesammelten Erdprobe aus Nigeria. Hinterlegungsnummer: DSM 4797. Hinterlegungstag: 2. September 1988.

5.) Sorangium(Polyangium)cellulosum Stamm "So ce 231", isoliert im April 1988 aus einer im April 1987 bei Didyma, Türkei, gesammelten Erdproben. Hinterlegungsnummer: DSM 5393. Hinterlegungstag: 2. Juni 1989.

6.) Sorangium(Polyangium)cellulosum Stamm "So ce 242", isoliert im Oktober 1988 aus einer im April 1988 bei Pozzuoli, Italien, gesammelten Erdprobe. Hinterlegungsnummer: DSM 5414. Hinterlegungstag: 19. Juni 1989.

Im folgenden wird für diese Stämme die Bezeichnung Sorangium cellulosum verwendet.

Das erfindungsgemässe Verfahren zur Herstellung eines "Soraphens" der Formel I ist gekennzeichnet durch aerobe Züchtung eines der Stämme Sorangium cellulosum "So ce 139", "So ce 170", "So ce 191", "So ce 192", "So ce 231" oder "So ce 242" oder eines von diesen ableitbaren Klon, Mutante etc. in einem geeigneten Nährmedium und Abtrennung des gebildeten "Soraphens".

In der europäischen Patentanmeldung EP-A-282,455 wird ein anderer Sorangium cellulosum-Mikroorganismus "So ce26" beschrieben, dessen Fermentation die hier weiter oben genannten Soraphene A und B liefert. Die vorliegende Erfindung bezieht sich nicht auf diese beiden Präparate und ihre alleinige Anwendung in Mitteln.

Die vorliegende Erfindung betrifft weitere wertvolle pflanzenmikrobizide Fermentationsprodukte, die durch den Mikroorganismus "So ce 26" (Hinterlegungsnummer NCIB 12 411) und oder durch die hierin beanspruchten Mikroorganismen "So ce 139", "So ce 170", "So ce 191", "So ce 192", "So ce 231" oder "So ce 242" produziert werden.

Allgemeine Verfahrensbedingungen für die Kultivierung der 6 Produktionsstämme

Die Sorangium cellulosum-Stämme lassen sich in geeigneten Nährmedien nach üblichen biologischen Methoden, z.B. in Schüttelkulturen oder in Fermentoren kultivieren. Die Fermentationstemperatur beträgt im Regelfall 10-40° C, bevorzugt 10-35° C und besonders bevorzugt bei etwa 30°-32° C, der pH-Wert beträgt 6-8, bevorzugt 7,4. Der Prozess verläuft aerob und unter sterilen Bedingungen.

Die Zusammenmsetzung des Nährmediums kann in grösseren Bereichen variieren. Für essentiell zu assimilierende Nährstoffe müssen eine Kohlenstoff- und eine Stickstoffquelle sowie eine Quelle für anorganische Mineralsalze, die P, S, Mg, K, Fe, Ca einschliessen, vorhanden sein.

Fermentierungsprozesse benutzen als C-Quellen bevorzugt Glucose, Stärke und Cellulose sowie deren Abbau-Produkte (z.B. Cellobiose) daneben auch Disaccharide, Glycerin, Essigsäure und andere. Als N-Quellen sind z.B. $NH_4$, $NO_3$ oder auch Peptone geeignet. Eine organische Verbindung als N-Quelle kann in der Regel nicht gleichzeitig die einzige C-Quelle und Energiequelle bei der Fermentierung sein.

Als Mineralsalze kommen Chloride, Nitrate, Sulfate, Carbonate und Phosphate der Elemente Na, K, $NH_4$, Mg, Fe und Ca in Betracht, daneben können Cu, Mn, Mo, Zn, Co und andere als Spurenelemente anwesend sein. Soweit möglich, können solche Salze auch an Ethylendiamintetraessigsäure (EDTA) gebunden vorliegen.

Die Mikroorganismus-Kultur wird in die Schüttelkultur oder in den Fermentor in einer Animpfmenge von 0,1-20 %, bevorzugt 0,5-10 %, ganz besonders bevorzugt 0,5-5 % (v/v), eingesetzt. Die Kulturdauer beträgt bei ca. 30° C etwa 2-7 Tage, grossvolumige Ansätze seltener bis zu 10 Tagen oder länger. Für grossvolumige Ansätze werden zweckmässigerweise einleitend kleinere Vorkulturen fermentiert. Die Verwendung der Sorangium cellulosum-Stämme ist auch in immobilisierter Form möglich, z.B. in Form trägerfixierter Zellen an Alginat.

Als ableitbarer Klon im obigen Sinne gilt jede Kultur, welche noch die für die Durchführung des erfindungsgemässen Verfahrens wesentlichen Merkmale des hinterlegten Klons aufweist, insbesondere eine Kultur, deren Mikroorganismen die gleichen Strukturgene wie die für die Bildung der Verbindung der Formel I ursächlichen Strukturgene der Stämme "So ce 139", "So ce 170", "So ce 191", "So ce 192", "So ce 231" und "So ce 242" enthalten. Als ableitbarer Klon gilt auch jede Kultur, deren Mikroorganismen ein wegen der Entartung des genetischen Codes mögliches Aequivalent der für die Bildung von Verbindung I ursächlichen Strukturgene der 4 Stämme enthalten. Als ableitbare Klon gilt insbesondere jede Kultur, die cellulosezersetzende Myxobakterien, vorzugsweise der Art Sorangium cellulosum, welche zur Produktion der Verbindung der Formel I befähigt sind, enthält. Der Begriff "ableitbarer Klon" der Stämme DSM 5397, DSM 4795, DSM 4796, DSM 4797, DSM 5393 und DSM 5414 schliesst auch alle Mutanten oder Rekombinanten ein, die zur Produktion von Verbindung I befähigt sind.

Die Züchtung erfolgt aerob, also beispielsweise in ruhender Oberflächenkultur oder vorzugsweise submers unter Schütteln oder Rühren mit Luft oder Sauerstoff in Schüttelkulturen oder in Fermentoren. Vorzugsweise kultiviert man stufenweise, d.h. man stellt zunächst eine oder mehrere Vorkulturen in flüssigem Nährmedium her, die dann in das eigentliche Produktionsmedium, z.B. in Verhältnis 1:20, überimpft werden.

Die Abtrennung des "Soraphens" erfolgt auf physiko-chemischem Wege mittels an sich bekannter Trennungsmethoden wie Filtrieren, besonders aber Lösungsmittel-Extraktion, und Chromatographie, vor

allem Adsorptions- und Verteilungschromatographie und gegebenenfalls Kristallisation.

Aus der wässrigen Fermentationsbrühe lassen sich die Verbindungen der Formel I mit lipophilen organischen Lösungsmitteln extrahieren, z.B. mit Ketonen wie Methylethylketon, Cyclohexanon; mit Alkoholen mittlerer Kettenlänge wie Isobutanol, Pentanol, Hexanol; mit Essigsäure-$C_1$-$C_6$-Alkylestern (Ethylacetat, Propylacetat, Butylacetat, Isobutylacetat u.a.); mit Toluol, Dichlormethan, 1,2-Dichlorethan, Chlorbenzol, Dichlorbenzol u.a.

Aus dem filtrierten Zellkuchen lassen sich die Verbindungen leicht mit Alkoholen oder Ketonen (z.B. Methanol, Ethanol, Aceton, Methylethylketon) extrahieren.

Aus dem jeweiligen Extrakt können dann durch Einengen und/oder Ausfällen Verbindungen der Formel I gewonnen werden, die sich durch fraktioniete Kristallisation oder andere chromatographische Trennverfahren in die Soraphene A bis ρ (= Rho) auftrennen lassen.

Vorteilhaft schliesst der Fermentationsvorgang zur Gewinnung der Verbindungen der Formel I mit der Zugabe eines Adsorberharzes ab, von dem die gewünschten Produkte aufgenommen werden, oder die Fermentation wird von Anfang an in Gegenwart eines solchen Adsorberharzes durchgeführt. Als Adsorberharze kommen vor allem neutrale organische Polymerstoffe in Frage, besonders nicht-ionische hydrophobe Adsorberharze, die zur lipophilen Extraktion geeignet sind. An diesem wird die Verbindung der Formel I fast quantitativ gebunden. Beispiele solcher Harze sind halbpolare Acrylester-Harze, unpolare Polystyrol/Divinylbenzol-Harze und besonders vernetztes Polystyrol. Solche Harze werden in Mengen von 0,1 bis 5 % (v/v) des Fermentations-Volumens zugegeben, bevorzugt 0,5 bis 2 % (v/v). Auch Aktivkohle kommt in Frage. Technisch besonders vorteilhaft sind Polystyrol-Harze wie z.B. XAD-1180 oder XAD-16 (Hersteller: Rohm and Haas), die in filtrierbarer Form vorliegen (Körner oder Granulate). Nach

Beendigung der Fermentation wird das Harz abfiltriert, mit Wasser gewaschen undmit Methanol oder Ethanol behandelt. Der alkoholische Extrakt wird eingeengt. Durch Zugabe von Diethylether, Ethylacetat oder Butylacetat kann die weitgehend auskristallisierende Verbindung IA (= Soraphen A) abgetrennt werden. Das Filtrat wird zur Gewinnung von restlichem Soraphen A, vor allem aber zur Gewinnung der restlichen Soraphene B bis σ (Sigma) chromatographisch gereinigt.

Die Erfindung betrifft die Verbindungen der Formel I in reiner Form bzw. in kristallisierter Form. Die Erfindung betrifft aber auch Biomassen, Rohextrakte oder Adsoberharze aus der Fermentation, die die Verbindung der Formel I enthalten und als solche oder in weiterformulierter Form zur Bekämpfung von Pflanzenkrankheiten verwendet werden können. Biomassen können auch als gemahlene oder gepresste Trockensubstanzen ("cake") weiterverwendet oder in den Handel gebracht werden.

Die beschriebenen Herstellungsverfahren einschliesslich aller Teilschritte sind ein Bestandteil der vorliegenden Erfindung.

Insbesondere betrifft die Erfindung ein Verfahren zur Herstellung von Soraphen der Formel I, dadurch gekennzeichnet, dass man einen der Stämme Sorangium cellulosum "So ce 139", "So ce 170", "So ce 191", "So ce 192", "So ce 231" und "So ce 242" oder einen Mikroorganismus, der die gleichen Strukturgene enthält wie die für die Bildung von Soraphen ursächlichen Strukturgene der genannten Stämme, in einem wässerigen, eine Kohlenstoff-und Stickstoffquelle sowie anorganische Salze enthaltenden Nährmedium aerob züchtet und die Soraphene der Formel I isoliert.

In erster Linie betrifft die Erfindung eine Ausführungsform des obengenannten Verfahrens, die dadurch gekennzeichnet, ist, dass man einen Soraphen bildenden Mikroorganismus aus der Gruppe der cellulosezersetzenden Myxobakterien züchtet.

Eine bevorzugte Ausführungsform des obengenannten Verfahrens ist dadurch gekennzeichnet, dass man einen der Stämme Sorangium cellulosum "So ce 139", "So ce 170", "So ce 191", "So ce 192", "So ce 231", "So ce 242" oder eine Soraphen bildende Mutante dieses ?Stammes züchtet.

Eine besonders bevorzugte Ausführungsform des obengenannten Verfahrens ist dadurch gekennzeichnet, dass man einen dieser gemäss Budapester Vertrag hinterlegten Stämme "So ce 139", "So ce 170", "So ce 191", "So ce 192", "So ce 231", "So ce 242" züchtet.

Vorzugsweise führt man die Fermentation unter den im Beispielteil beschriebenen Bedingungen durch.

Mikroorganismen, die die gleichen für die Bildung von Soraphen ursächlichen Strukturgene enthalten wie die 6 Stämme, können z.B. durch Genmanipulation künstlich geschaffen werden, indem man die entsprechenden Strukturgene aus den 6 Stämmen isoliert und an geeigneter Stelle in das Genmaterial eines geeigneten anderen Mikroorganismus einbaut. Geeignete Mikroorganismen sind solche, in die man die betreffenden Strukturgene nicht nur einbauen kann, sondern in denen diese Strukturgene auch exprimiert werden und in denen das gebildete Soraphen nicht wieder abgebaut, sondern vorzugsweise in die Fermentationsbrühe ausgeschieden wird. Solche geeigneten Mikroorganismen sind in erster Linie andere Stämme der Myxobakterien, insbesondere solche der Art Sorangium cellulosum, sofern sie die obengenannten Strukturgene nicht bereits besitzen.

Soraphen bildende Mutanten können zum Beispiel unter der Einwirkung von Ultraviolett- oder Röntgenstrahlen oder von chemischen Mutagenen, z.B. N-Methyl-N′-nitro-N-nitroso-guanidin, erzeugt und durch Selektion nach ihren spezifischen Eigenschaften in an sich bekannter Weise isoliert werden. Weitere Verfahrensmassnahmen zur Herstellung von Mutanten und Rekombinanten eines Mikroorganismus sind dem Fachmann bekannt und geläufig.

Die Erfindung betrifft auch ein Verfahren zur Herstellung eines Fermentationsgutes, das Soraphen in nachweisbarer Menge enthält und das dadurch gekennzeichnet ist, dass man einen der Stämme Sorangium

cellulosum "So ce 139", "So ce 170", "So ce 191", "So ce 192", "So ce 231", "So ce 242" oder eine von diesen Stämmen ableitbaren Klon in einem Nährmedium züchtet und Soraphen in geeigneter Form gewinnt. Solche Kulturbrühen lassen sich als solche oder in konzentrierter Form, gegebenenfalls unter Zusatz von weiteren Trägerstoffen und/oder Verteilungsmitteln als Mittel gegen pflanzenpathogene Mikroorganismen einsetzen und sind daher ein wichtiger Teil vorliegender Erfindung.

Im engeren Sinne ist das Verfahren dadurch gekennzeichnet, dass man einen der Mikroorganismen Sorangium cellulosum "So ce 139","So ce 170", "So ce 191", "So ce 192", "So ce 231" oder "So ce 242" in einem Kulturmedium enthaltend mindestens je eine assimilationsfähige C-Quelle und N-Quelle sowie entsprechende anorganische Salze, bei 10-40°C, bevorzugt bei 10-35°C, in Anwesenheit oder Abwesenheit eines Adsorberharzes kultiviert, dann die Kulturbrühe bzw. das abfiltrierte Adsorberharz mit einer geeigneten Lösungsmittelphase extrahiert, die erhaltene Lösung einengt und den verbleibenden Rückstand, sofern gewünscht, durch Chromatographie und/oder Umkristallisation reinigt.

Stammkulturen und morphologische Beschreibung:

Stammkulturen werden gehalten als Plattenkulturen auf VY/2-Agar (0,5 % Bäckerhefe nach Frischgewicht; 0,1 % $CaCl_2$; 1,5 % Agar; pH 7,2) oder auf Filterpapier (= Cellulosequelle) über ST21-Agar (0,1 % $KNO_3$; 0,1 % $MgSO_4.7H_2O$; 0,1 % $CaCl_2$; 0,1 % $K_2HPO_4$; 0,01 % $MnSO_4.7H_2O$; 0,02 % $FeCl_3$; 0,002 % Hefe-extrakt; Standard-Spurenelementlösung[1]; 1 % Agar). Die Platten werden bei 30° bebrütet.

[1] 0,02-0,5 mg Mn-, Mo-, Cu-, Co- und/oder Zn-Salz pro Liter [R.Y. Stanier et al. "General Microbiology" 4th Ed., p. 36 (1976)];
oder
500 mg EDTA, 300 mg $FeSO_4 \cdot 7H_2O$, 3 mg $MnCl_2 \cdot 4H_2O$, 5 mg $CoCl_2 \cdot 6H_2O$, 1 mg $CuCl_2 \cdot 2H_2O$, 2 mg $NiCl_2 \cdot 6H_2O$, 3 mg $Na_2MoO_4 \cdot 2H_2O$, 5 mg $ZnSO_4 \cdot 7H_2O$, 2 mg $H_3BO_3$ pro Liter dest. Wasser (ca. pH 4) [G. Drews "Mikrobiolog. Praktikum", 4. Auflage, S. 11, Springer Verlag Berlin, Heidelberg, New York, Tokyo [1983]

Auf beiden Medien bilden die Organismen Schwarmkolonien, die sich langsam über das Substrat hin ausbreiten. Im einzelnen beobachtet man deutliche Unterschiede zwischen den 6 Stämmen.

1) "So ce 139" (DSM 5397): Auf Filterpapier über ST 21 Agar bleibt die Schwarmkolonie weitgehend auf das Filterpapier beschränkt. In älteren Kulturen reisst der Agar am Filterrand auf, und Schwarm und Agar rollei sich ein. Das Filterpapier wird vollständig abgebaut, an seiner Stelle findet man ein dichtes Feld von schwarzbraunen Fruchtkörpern. Diese sind aus kleinen Sporangiolen von 15-30 µm Durchmesser zusammengesetzt, die ihrerseits zu kleinen Paketen bis zu ausgedehnten ungegliederten Massen verbacken sind. Die vegetativen Zellen sind schlanke zylindrische Stäbchen mit stumpf abgerundeten Enden und messen 0.7-0.9 x 2.5-4 µm. Im Phasenkontrastmikroskop erscheinen sie dunkel, oft mit hellen Polkörnchen. Auf VY/2-Agar bilden sich sehr grosse Schwarmkolonien mit farblosen bis orange radialen Adern und vielen schwarzbraunen Fruchtkörpern von ähnlichem Bau wie oben beschrieben. Die Hefezellen des Nährmediums werden kaum angegriffen. Die vegetativen Zellen erscheinen etwas derber und sind um 1 µm dicker.

2) "So ce 170" (DSM 4795): Auf Filterpapier über ST 21 Agar dringt der Schwarm weit über das Filter hinaus auf die Agaroberfläche vor. Der Agar reisst später tief auf, und die Schwarmkolonie rollt sich ein. Auf dem Filterpapier können sich Fruchtkörper entwickeln: Diese bestehen aus kleinen dickwandigen dunkelbraunen Sporangiolen, von meist 20-30 µm Ø, die in meist scharf begrenzten länglichen Paketen von etwa 50-200 µm Ø dicht zusammengedrängt sind. Stellenweise können zusammenhängende ausgedehnte Aggregate von Fruchtkörpern entstehen. Auf VY/2-Agar bilden sich knapp mittelgrosse, dickschleimige, intensiv orange Schwarmkolonien von meist 1-2 cm Ø. Die Hefezellen werden langsam abgebaut. Auch hier reisst der Agar stellenweise tief auf, so dass der Schwarm ganz aus der Platte herausfallen kann. Auf der Agaroberfläche bilden sich oft orange, hellbraune bis dunkelbraune Fruchtkörper von ähnlichem Bau wie oben. Die vegetativen Zellen sind derbe, im Phasenkontrastmikroskop dunkle Stäbchen mit breit abgerundeten Enden und messen gewöhnlich 0,8-1,0 x 2-5 µm. Der Stamm baut auch sehr effizient Chitin ab.

3) "So ce 191" (DSM 4796) wächst auf Filterpapier über ST21-Agar, ohne auf die Agaroberfläche überzugreifen. Auch in diesem Fall kann der Agar rings um das Filterpapier tief aufreissen und die Schwarmkolonie sich einrollen. Auf VY/2-Agar entwickeln sich grosse Schwärme von 6-8 cm Ø, mit feinen bis kräftigen radialen Adern, Die Hefezellen werden kaum angegriffen. Die zylindrischen vegetativen Stäbchen haben breit abgerundete Enden, sind im Phasenkontrastmikroskop dunkel, oft ziemlich lang und messen meist 0,7-0,9 x 3-8 µm. Der Stamm bildet in Reinkultur unter den genannten Kulturbedingungen keine Fruchtkörper. Der Stamm baut effizient Chitin ab.

4) "So ce 192" (DSM 4797) breitet sich bei Kultur auf Filterpapier über ST21-Agar weit über die Agaroberfläche hin aus. Der Agar kann tief aufreissen, die Schwarmkolonie sich einrollen. Auf der Agaroberfläche, besonders aber auf auf dem Filterpapier entwickeln sich viele leuchtend orange gefärbte Fruchtkörper, bestehend aus winzigen Sporangiolen von meist 10-20 µm Ø in scharf begrenzten Paketen von meist 50-500 µm Ausdehnung. Auf VY/2-Agar entwickeln sich mittelgrosse kräftige Schwärme von 2-3 cm Ø, mit radialen Adern und vielen intensiv braunorange gefärbten Fruchtkörpern in und auf dem Agar von ähnlichem Bau, wie oben beschrieben. Die vegetativen Zellen sind zylindrische dunkle Stäbchen mit breiten Enden und messen meist 0,7-0,9 x 3-5 µm. Die Hefezellen werden nicht abgebaut, dagegen baut der Stamm sehr effizient Chitin ab.

5) "So ce 231" (DSM 5393) geht in Kulturen auf Filterpapier über ST 21 Agar nicht auf die Agarplatte über. Der Agar kann am Filterrand aufreissen, doch rollen sich die Kolonien nicht ein. Das Filterpapier wird vollständig zerstört, an seiner Stelle findet man später ein sehr dichtes Feld von intensiv braunorange Fruchtkörpern. Dies sind ähnlich gebaut wie bei den Anderen Stämmen, doch sind die Sporangiolen häufig in Bändern angeordnet. Auf VY/2-Agar entwickeln sich sehr grosse Schwärme mit radialen Bündeln von feinen Adern. Lokal entwickeln sich leuchtend orangebraune Fruchtkörper. Die schlanken vegetativen Stäbchen sind sehr zierlich und messen 0.6-0.8 x 2.5-6 µm. Die Hefezellen des Nährbodens werden langsam lysiert. Der Stamm baut auch sehr effizient Chitin ab.

6) "So ce 242" (DSM 5414) dringt auf Filterpapier über ST 21 Agar vor und bildet dort auch Früchtkörper. Der Agar reisst am Rand des Filterpapiers tief auf. Das Filterpapier wird völlig abgebaut und durch eine dichte Masse aus schwarzbraunen Fruchtköpern ersetzt. Die Sporangiolen sind in diesem oft in Ketten angeordnet. Auf VY/2-Agar entwickeln sich sehr grosse Schwärme mit feinen radialen Adern. Der Agar reisst im Schwarmbereich an vielen Stellen krallenartig auf. Ferner entstehen reichlich Fruchtkörper. Die Hefezellen im Substrat werden langsam abgebaut. Die vegetativen Zellen messen 0.7-1.0 µm x 2-4µm. Der Stamm ist ein sehr effizienter Chitinzersetzer.

Die Stämme produzieren Substanzen, welche das Wachstum zahlreicher Hefen und Hyphenpilze hemmen. Chemisch handelt es sich bei den Hemmstoffen nicht nur um Soraphene, deren Strukturen, wie unten angegeben, bereits aufgeklärt sind. Diese Gemische macrocyclischer Verbindungen vom Soraphen-Typ erscheinen in Flüssigkulturen im Kulturüberstand, können aber auch aus den Zellen isoliert werden. Die Produktion der Antibiotika erfolgt während der logarithmischen bis in die stationäre Wachstumsphase hinein.

Alle 6 Stämme müssen an das Wachstum in Flüssigmedien adaptiert werden. Sie wachsen zunächst in Form kleiner, fester, orangefarbener Knöllchen und bilden erst nach vielen Uebertragungsschritten von Flüssigmedium zu Flüssigmedium allmählich mehr oder weniger homogene Zellsuspensionen.

Biologische Charakterisierung der Stämme "So ce 139", "So ce 170", "So ce 191", "So ce 192", "So ce 231" und "So ce 242"

Cellulose-Abbau : positiv
Glucose-Abbau : positiv
Stärke-Abbau : positiv
$NH_4$ als N-Quelle: positiv
$NO_3$ als N-Quelle: positiv

Tabelle 1

Beispiele für Organismen, welche durch Kulturüberstände der Stämme So ce 139, So ce 170, So ce 191, So ce 192, So ce 231 und So ce 242 im Wachstum gehemmt werden

| Hefen: | Bewertung* |
|---|---|
| Debaryomyces hansenii | starke Hemmung |
| Nematospora coryli | starke Hemmung |
| Candida albicans | starke Hemmung |
| Saccharomyces cerevisiae | mittelstarke Hemmung |
| Rhodotorula glutinis | mittelstarke Hemmung |
| Hansenula anomala | mittelstarke Hemmung |
| Nadsonia fulvescens | keine Hemmung |
| Torulopsis glabrata | keine Hemmung |
| Schizosaccharomyces pombe | keine Hemmung |
| Hyphenpilze | |
| Alternaria solani | starke Hemmung |
| Pythium debaryanum | starke Hemmung |
| Mucor hiemalis | starke Hemmung |
| Rhizopus arrhizus | schwache Hemmung |

*Die Hemmwerte beziehen sich auf eine durchschnittliche Kulturen-Charge und sind als Verhältniswerte zueinander aufzufassen.

Die Erfindung betrifft die Soraphene C bis $\sigma$ der Formel I in reiner Form. Die Erfindung betrifft aber auch Biomassen, Rohextrakte oder Adsorberharze aus der Fermentation, die die Soraphene C bis $\sigma$ der Formel I

enthalten und als solche oder in weiterformulierter Form zur Bekämpfung von Pflanzenkrankheiten verwendet werden können. Biomassen können auch als gemahlene oder gepresste Trockensubstanzen ("cake") weiterverwendet oder in den Handel gebracht werden.

Die beschriebenen Herstellungsverfahren einschliesslich aller Teilschritte sind ein Bestandteil der vorliegenden Erfindung.

Ueberraschenderweise wurde nun gefunden, dass Verbindungen der Formel I ein sehr günstiges biozides Spektrum gegen phytopathogene Mikroorganismen, insbesondere gegen Pilze, aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Als Mikrobizide sind die Wirkstoffe der Formel I z.B. gegen die den folgenden Klassen angehörenden phytopathogenen Pilze wirksam: Fungi imperfecti (z.B. insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. insbesondere Venturia und Erysiphe, ferner Podosphaera, Monilinia, Uncinula) und der Oomyceten (z.B. Phytophthora, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente eine oder mehrere der Soraphene C bis $\sigma$ der Formel I enthalten, insbesondere pflanzenschützende Mittel, sowie deren Verwendung auf dem Agrarsektor oder verwandten Gebieten.

Eingeschlossen ist auch ein Verfahren zur Behandlung von Pflanzen, das sich durch Applikation der neuen Verbindungen Soraphen C bis $\sigma$ der Formel I bzw. der entsprechenden neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Ananas, Zuckerrohr, Tee, Pfeffer, Weinreben, Hopfen, Bananen- und Naturkautschukgewächse sowie Zierpflanzen (Compositen). Diese Aufzählung stellt keine Limitierung dar.

Wirkstoffe der Formel I werden üblicherweise in Form von Zusammensetzung verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Dieses Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Granulat oder ein entsprechendes Pulver kann auch die Trockenmasse der aus dem Fermentor anfallenden Biomasse oder das aus der Fermentationsbrühe abgesiebte und mit den Wirkstoffen der Formel I beladene Adsorberharz sein. Die Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 2 kg Aktivsubstanz (AS) je ha, bevorzugt bei 50 g bis 500 g AS/ha.

Die Formulierungen d.h. die den Wirkstoff der Formel I und gegebenenfalls einen festen oder flüssigen Zusatzstoff enthaltenden Mittel werden in bekannter Weise hergestellt.

Als Lösungsmittel kommen in Frage: Aromatische und aliphatische Kohlenwasserstoffe, wie z.B. Xylolgemische, Cyclohexan oder Paraffine; dann Alkohole und Glykole sowie deren Ether und Ester, wie Ethanol, Ethylenglykol, Ethylenglykolmonomethyl- oder -ethylether, Essigsäureester; Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Häufiger werden jedoch sog. synthetische Tenside verwendet, insbesondere Alkansulfonate, Fettalkoholsulfate, sulfonierte Benzimidazolderivate oder Alkylsulfonate.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als weitere Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood New Jersey, 1980 Sisley and Wood, "Encyclopedia of Surface Active Agents", Chemical Publishing Co., Inc. New York, 1980.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin oder Lysolecithin.

Die agrochemischen Zubereitungen enthalten in der Regel 0,1 bis 95 % Wirkstoff der Formel I, 99,9 bis 5 % eines festen oder flüssigen Zusatzstoffes und 0 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselben einzuschränken.

## 1. Herstellungsbeispiele

### Beispiel H-1: Herstellung der Verbindungen der Formel I in Gegenwart eines Adsorberharzes

Das Verfahren wird in einem Fermentationsvolumen von 1000 Litern unter Zugabe von 0,5 % (v/v) Adsorberharz XAD-1180 (Rohm und Haas) durchgeführt. Die Verfahrensbedingungen für diesen Ansatz entsprechen denen des nachfolgenden Beispiels H-2. An Stelle des hier verwendeten Stammes So ce 26 aus der EP-A-282,455 kann auch einer der anderen 6 genannten Stämme vorliegender Erfindung eingesetzt werden.

Nach Beendigung der Fermentation wird der Polymerträger abgesiebt, in eine Glassäule gespült, mit 3 Bettvolumen Wasser gewaschen und mit 4 Bettvolumen Methanol eluiert. Das Eluat wird im Vakuum zur Trockne eingeengt.

Rohextraktgewicht: 165 g.

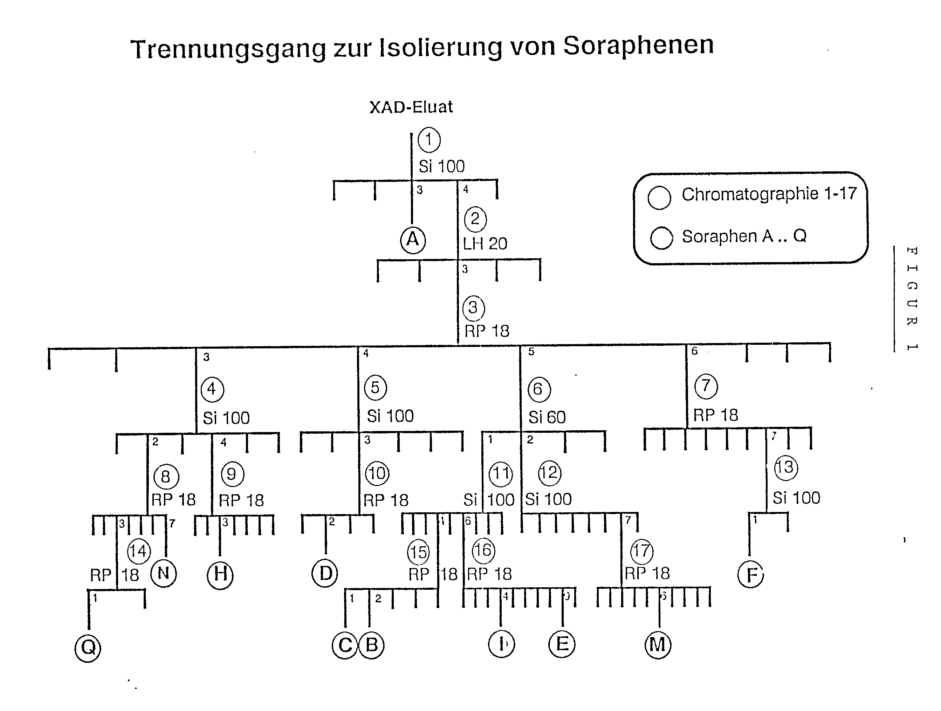

Die weitere chromatographische Aufreinigung folgt dem Schema der beigefügten Figur 1, deren einzelne Trennungsschritte unter folgenden Bedingungen ablaufen. Die Ausbeuten sind in Klammern angegeben. (h = Stunde)

### Trennungsgang zur Isolierung von Soraphen A bis Q

1 Kieselgeltrennung; Säule: 100 mm Ø; 45 cm Länge;

Sorbens: Lichroprep Si 100; 40-63 μm (Hersteller: Merck)
Laufmittel: Dichlormethan/Aceton in Stufen 98/2, 95/5, 93/7, 90/10, 50/50; je Stufe 2 Liter
Es werden 5 Fraktionen genommen.
Die Fraktion 3 enthält Soraphen A.
Die Fraktion 4 (25 g) wird weiter aufgereinigt.

2 Gelchromatographie; Säule: 60 mm ∅; 100 cm Länge;
Sorbens: Sephadex LH-20 (Hersteller: Pharmacie LKB GmbH)
Laufmittel: Methanol
Es werden 5 Fraktionen genommen.
Die Fraktion 3 (17 g) wird weiter aufgereinigt.

3 Reversed-Phase-Trennung; Säule: 76 mm ∅; 70 cm Länge;
Sorbens: HDSIL RP-18; 18-60-60 35-70 μm (Hersteller: Labomatic)
Laufmittel: Methanol/Wasser 40/60 lin. Gradient in 2 h nach Methanol.
Es werden 9 Fraktionen genommen.
Die Fraktionen 3 (1,3 g), 4 (1,5 g), 5 (3,5 g) und 6 (1,6 g) werden weiter aufgereinigt.

4 Kieselgeltrennung; Säule: 40 mm ∅; 30 cm Länge;
Sorbens: Lichrosorb Si 100; 7 μm (Hersteller: Merck)
Laufmittel: Dichlormethan/Hexan 1/1 + 2 % Methanol
Es werden 6 Fraktionen genommen.
Die Fraktionen 2 (500 mg) und 4 (88 mg) werden weiter aufgereinigt.

5 Kieselgeltrennung; Säule: 40 mm ∅; 30 cm Länge;
Sorbens: Lichrosorb Si 100; 7 μm (Hersteller: Merck)
Laufmittel: Dichlormethan/Hexan 1/1 + 2 % Methanol
Es werden 6 Fraktionen genommen.
Die Fraktion 3 (500 mg) wird weiter aufgereinigt.

6 Kieselgeltrennung; Säule: 35 mm ∅; 45 cm Länge;
Sorbens: Lichroprep Si 60; 40-63 μm (Hersteller: Merck)
Laufmittel: Dichlormethan lin. Gradient in 1 h nach Dichlormethan/Aceton 1/1.
Es werden 6 Fraktionen genommen.
Die Fraktionen 1 (2,4 g) und 2 (400 mg) werden weiter aufgereinigt.

7 Reversed-Phase-Trennung; Säule: 35 mm ∅; 45 cm Länge;
Sorbens: HDSIL RP-18; 18-30-60 25-40 μm (Hersteller: Labomatic)
Laufmittel: Methanol/Wasser 80/20.
Es werden 9 Fraktionen genommen.
Die Fraktion 7 (1,4 g) wird weiter aufgereinigt.

8 Reversed-Phase-Trennung: Säule: 20,5 mm ∅; 25 cm Länge;
Sorbens: Nucleosil 100-7 $C_{18}$; 7 μm (Hersteller: Macherey Nagel)
Laufmittel: Methanol/Wasser 70/30.
Es werden 7 Fraktionen genommen. Die Fraktion 3 (15 mg) wird weiter aufgereinigt.
Die Fraktion 7 enthält Soraphen N (18 mg).

9 Reversed-Phase-Trennung; Säule: 20,5 mm ∅; 25 cm Länge;
Sorbens: Nucleosil 100-7 $C_{18}$; 7 μm (Hersteller: Macherey Nagel)
Laufmittel: Methanol/Wasser 65/35.
Es werden 7 Fraktionen genommen.
Die Fraktion 3 enthält Soraphen H (7 mg).

10 Reversed-Phase-Trennung; Säule: 20,5 mm ∅; 25 cm Länge;
Sorbens: HDSIL 100-$C_{18}$-10 μm (Hersteller: Labomatic)
Laufmittel: Methanol/Wasser 65/35.
Es werden 4 Fraktionen genommen.
Die Fraktion 2 enthält Soraphen D (108 mg).

11 Kieselgeltrennung; Säule: 40 mm ∅; 30 cm Länge;
Sorbens: Lichrosorb Si 100; 7 μm (Hersteller: Merck)
Laufmittel: Dichlormethan/Hexan 1/1 + 1 % Methanol.
Es werden 9 Fraktionen genommen.
Die Fraktionen 4 (530 mg) und 6 (280 mg) werden weiter aufgereinigt.

12 Kieselgeltrennung; Säule: 40 mm ∅; 30 cm Länge;
Sorbens: Lichrosorb Si 100; 7 μm (Hersteller: Merck)
Laufmittel: Dichlormethan/Hexan 1/1 + 1 % Methanol.
Es werden 8 Fraktionen genommen.
Die Fraktion 7 (290 mg) wird weiter aufgereinigt.

13 Kieselgeltrennung; Säule: 20.5 mm ∅; 25 cm Länge;
Sorbens: Nucleosil Si 100-7; 7 μm (Hersteller: Macherey Nagel)
Laufmittel: tert-Butylmethylether/Hexan 1/2 + 1 % Methanol.
Es werden 2 Fraktionen genommen.
Die Fraktion 1 (325 mg) enthält Soraphen F.

14 Reversed-Phase-Trennung; Säule: 20,5 mm ∅; 25 cm Länge;
Sorbens: Nucleosil 100-7 $C_{18}$; 7 µm (Hersteller: Macherey Nagel)
Laufmittel: Methanol/Wasser 68/32.
Es werden 2 Fraktionen genommen.
Die Fraktion 1 enthält Soraphen Q (3 mg).

15 Reversed-Phase-Trennung; Säule: 20,5 mm ∅; 25 cm Länge;
Sorbens: Nucleosil 100-7 $C_{18}$; 7µm (Hersteller: Macherey Nagel)
Laufmittel: Methanol/Wasser 66/34.
Es werden 4 Fraktionen genommen.
Die Fraktion 1 enthält Soraphen C (21 mg).
Die Fraktion 2 enthält Soraphen B (230 mg).

16 Reversed-Phase-Trennung; Säule: 20,5 mm ∅; 25 cm Länge;
Sorbens: Nucleosil 100-7 $C_{18}$; 7 µm (Hersteller: Macherey Nagel)
Laufmittel: Methanol/Wasser 65/35.
Es werden 10 Fraktionen genommen.
Die Fraktion 4 enthält Soraphen J (18 mg).
Die Fraktion 9 enthält Soraphen E (96 mg).

17 Reversed-Phase-Trennung; Säule: 20,5 mm ∅; 25 cm Länge;
Sorbens: Nucleosil 100-7 $C_{18}$; 7 µm (Hersteller: Macherey Nagel)
Laufmittel: Methanol/Wasser 70/30.
Es werden 10 Fraktionen genommen.
Die Fraktion 6 enthält Soraphen M (98 mg).

Physikochemische Charakterisierung der Soraphene C bis Q

Soraphen C
$C_{28}H_{42}O_8$
Mz 506
IR (Film)
3411; 2939; 2831; 1725; 1461; 1382; 1266; 1230; 1187; 1152; 1098; 1068; 1023; 988; 975.
$^{13}C$-NMR-Verschiebungen ($CDCl_3$; δ in ppm)
10.3; 11.7; 12.5; 23.0; 26.0; 29.4; 35.2; 35.6; 35.8; 46.2; 57.3; 57.6; 68.8; 72.5; 74.6; 74.9; 76.1; 83.7; 99.4; 125.0; 126.2; 126.2; 128.2; 128.6; 137.3; 141.0; 170.6.
HPLC $R_t$ = 8,6 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen D
$C_{27}H_{42}O_8$
Mz 494
IR (Film)
3411; 2937; 2831; 1733; 1461; 1432; 1382; 1359; 1328; 1268; 1208; 1195; 1096; 1050; 988, 933.
$^{13}C$-NMR-Verschiebungen ($CDCl_3$; δ in ppm)
10.7; 14.4; 22.8; 24.3; 25.0; 27.5; 27.7; 31.6; 35.3; 35.6; 43.2; 57.5; 58.6; 69.1; 70.8; 71.2; 74.8; 80.7; 82.6; 97.7; 126.4; 126.4; 128.0; 128.6; 128.6; 141.0; 168.6.
HPLC $R_t$ = 7,4 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen E
$C_{29}H_{46}O_9$
Mz 538
IR (Film)
3398; 2939; 2829; 1725; 1461; 1430; 1380; 1266; 1235; 1191; 1154; 1102; 1044; 971.
$^{13}C$-NMR-Verschiebungen ($CDCl_3$; δ in ppm)
10.3; 10.5; 11.5; 23.0; 25.0; 29.5; 30.0; 35.3; 35.5; 40.2; 46.3; 56.4; 57.4; 58.0; 69.0; 71.3; 71.7; 75.0; 76.3; 80.2; 81.1; 99.9; 126.5; 126.5; 128.1; 128.6; 140.9; 170.9.
HPLC $R_t$ = 11,8 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen F
$C_{29}H_{46}O_8$
Mz 522
IR (Film)
3394; 2937; 2827; 1729; 1710; 1461; 1382; 1326; 1314; 1266; 1253; 1232; 1189; 1154; 1104; 1075; 1048; 1021; 994; 971; 907.
$^{13}$C-NMR-Verschiebungen ($CDCl_3$; δ in ppm)
10.4; 11.7; 14.1; 23.0; 24.2; 24.2; 28.3; 28.8; 33.3; 34.1; 35.2; 45.7; 57.4; 57.4; 57.9; 69.0; 70.1; 75.9; 76.3; 80.8; 82.1; 99.6; 126.6; 126.6; 128.2; 128.6; 128.6; 140.4; 171.8.
HPLC $R_t$ = 8,1 min
Säule: 4 x 250 mm Nucleosil 100-7 $C_{18}$, Macherey Nagel;
Fluss: 1,5 ml/min; Laufmittel: Methanol/Wasser 75/25;
Detektor: UV 210 nm.

Soraphen H
$C_{28}H_{42}O_9$
Mz 522
IR (Film)
3444; 2935; 2867; 2833; 1723; 1459; 1409; 1382; 1334; 1270; 1230; 1179; 1156; 1104; 1069; 996; 971.
$^{13}$C-NMR-Verschiebungen ($CDCl_3$; δ in ppm)
10.9; 11.6; 14.4; 23.4; 25.0; 28.6; 35.6; 36.3; 45.6; 57.7; 57.8; 67.5; 68.7; 69.2; 73.9; 73.9; 76.1; 81.7; 100.1; 122.9; 126.0; 126.0; 128.2; 128.7; 128.7; 138.0; 140.6; 171.5.
HPLC $R_t$ = 7,0 min
Säule: 4 x 250 mm Nucleosil 100-7 $C_{18}$, Macherey Nagel;
Fluss: 1,5 ml/min; Laufmittel: Methanol/Wasser 65/35;
Detektor: UV 210 nm.

Soraphen J
$C_{28}H_{44}O_8$
Mz 508
IR (Film)
3394; 2939; 2829; 1729; 1461; 1380; 1313; 1270; 1187; 1158; 1100; 1042; 987.
$^{13}$C-NMR-Verschiebungen ($CDCl_3$; δ in ppm)
11.5; 13.8; 14.3; 22.5; 24.1; 25.6; 25.8; 27.7; 31.6; 35.2; 36.3; 49.3; 57.3; 59.4; 69.6; 70.5; 72.6; 75.7; 81.0; 84.6; 98.2; 126.3; 126.3; 128.1; 128.7; 128.7; 141.3; 172.5.
HPLC $R_t$ = 8,1 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen M
$C_{28}H_{44}O_9$
Mz 524
IR (Film)
3396; 2939; 2831; 1725; 1461; 1380; 1270; 1235; 1191; 1154; 1075; 1048; 994; 971; 898; 850;
$^{13}$C-NMR-Verschiebungen ($CDCl_3$; δ in ppm)
9.0; 10.4; 11.5; 23.1; 25.3; 28.3; 29.3; 35.1; 35.7; 40.3; 46.5; 57.2; 57.9; 68.8; 68.8; 71.9; 72.9; 74.4; 76.3; 83.8; 99.6; 126.3; 126.3; 128.0; 128.5; 128.5; 141.2; 171.0.
HPLC $R_t$ = 8,5 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen N
$C_{29}H_{44}O_9$
Mz 536
IR (Film)
3444; 2966; 2939; 1731; 1461; 1380; 1309; 1268; 1224; 1164; 1098;
$^{13}$C-NMR-Verschiebungen ($CDCl_3$; δ in ppm)
13.8; 13.9; 23.3; 23.4; 24.2; 26.3; 26.7; 29.5; 32.1; 36.3; 50.3; 54.5; 57.6; 68.1; 75.2; 78.4; 79.9; 105.7; 125.8; 125.8; 127.5; 128.4; 128.4.
$^{1}$H-NMR-Verschiebungen ($CDCl_3$; δ in ppm)
0,9 d; 1.05 d; 1.24 d; 5.23 d; 5.85 dd.
$HPLC_t$ = 13,4 min
Säule: 4 x 250 mm Nucleosil 100-7 $C_{18}$, Macherey Nagel;

Fluss: 1,5 ml/min; Laufmittel: Methanol/Wasser 70/30;
Detektor: UV 210 nm.

Soraphen Q
$C_{28}H_{44}O_8$
Mz 508
IR (Film)
3448; 2939; 2831; 1733; 1459; 1382; 1330; 1270; 1203; 1098; 1048; 987
$^{13}$C-NMR-Verschiebungen ($CDCl_3$; δ in ppm)
11.5; 14.5; 23.1; 24.9; 26.2; 27.1; 27.6; 31.7; 35.5; 35.9; 39.5; 50.7; 57.4; 58.7; 69.5; 71.3; 71.6; 74.6; 79.4; 81.9; 102.2; 126.6; 126.6; 127.9; 128.4; 128.4; 137.9; 168.0.
HPLC $R_t$ = 7,7 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Beispiel H-2: Herstellung der Verbindungen der Formel I

a) Vorkultur:

Die Vorkultur wird im 2 Liter-Kolben mit je 500 ml eines Kulturmediums (enthaltend 0,1 % Pepton aus Casein, 0,5 % Glucose, 0,05 % $CaCl_2 \cdot H_2O$, 0,05 % $MgSO_4 \cdot 7H_2O$) bei pH 7,4 ohne Puffer (oder mit 50 mM HEPES-Puffer[2]) bei 160 Upm und 30°C auf dem Schütteltisch angezogen. Der günstigste Zeitpunkt zur Uebertragung in die Fermentor-Kultur wird nach 2-3 Tagen (obere log-Phase) erreicht. An Stelle des hier verwendeten Stammes So ce 192 kann auch einer der anderen 5 Stämme vorliegender Erfindung oder der Stamm So ce 26 gemäss EP-A-282,455 eingesetzt werden.

([2] HEPES ist das K- und/oder Na-Salz der 4-(2-Hydroxyethyl)-piperazin-1-ethansulfonsäure)

b) Fermentation:

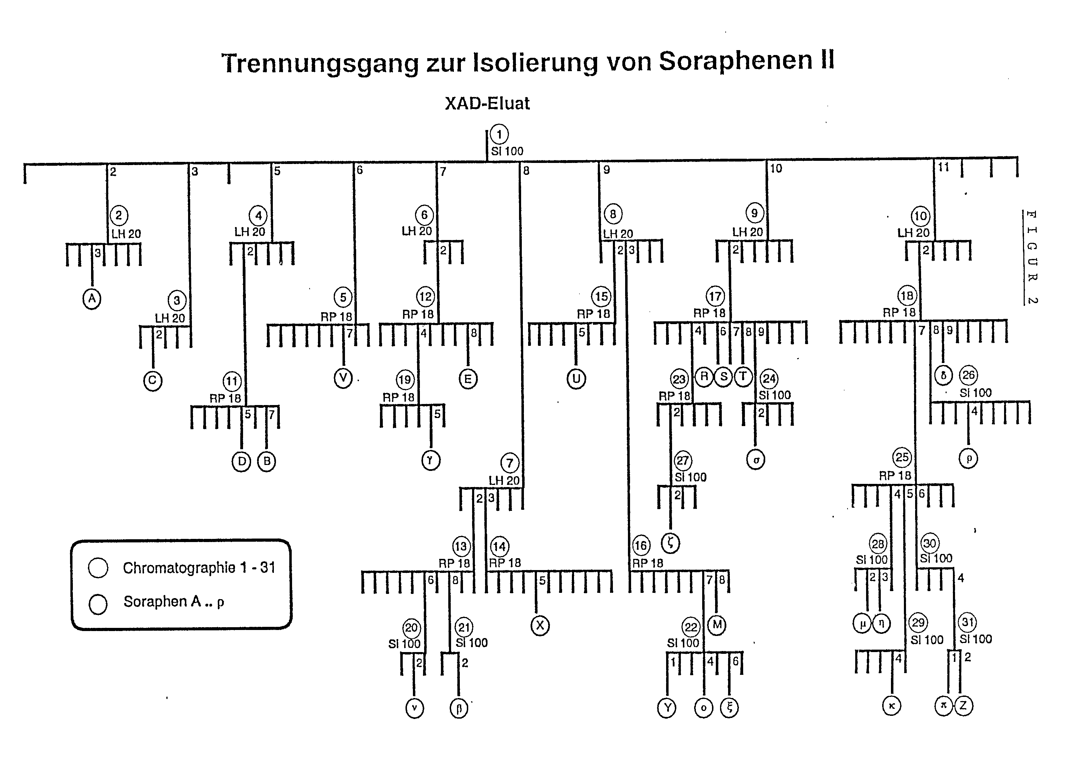

Ein 70 Liter-Fermentor der Firma Giovanola Frères, Monthey, Schweiz, mit 60 Litern des gleichen Kulturmediums wird mit 10 Litern Vorkultur beimpft. Die Fermentation erfolgt bei 30-32°C. Die Rührgeschwindigkeit ist 500 Upm, die Belüftungsrate 0,12 Liter pro Liter Medium und Stunde. Die Fermentationsdauer beträgt 7-14 Tage. Es wird darauf geachtet, dass der pH-Wert nicht unter 7,0, insbesondere nicht unter 6,2, fällt. Die gebildeten macrocyclischen Verbindungen der Formel I befinden sich teils im Kulturüberstand, teils in den Zellen. Sie können aus den Zellen mit Alkoholen oder Ketonen (z.B. Aceton), aus dem Kulturüberstand mit Ethylacetat oder Butylacetat extrahiert werden. Die Fermentationsbrühe wird dann z.B. fünfmal mit je 2 Litern Ethylacetat je 5 min. ausgeschüttelt. Diese vereinigten Extrakte werden zweimal mit Wasser gewaschen und im Vakuum eingeengt. Das zurückbleibende dunkle Oel kann nach der in der Figur 2 wiedergegebenen Methode aufgetrennt werden.

Vorteilhafter jedoch werden die gebildeten Verbindungen der Formel I nach Beendigung der Fermentation mit Adsorberharz aus dem Fermentationsvolumen entfernt. Hierzu gibt man 0,5 % v/v feinkörniges Harz (z.B. XAD-1180 oder XAD-16) in die Fermentorbrühe und rührt 4 Std. lang, wonach die Verbindungen der Formel I vollständig am Harz gebunden sind. Nach Abtrennung der Fermentorbrühe durch Absieben wird das Harz in eine Glassäule gespült, mit 3 Bettvolumen Wasser gewaschen und mit 4 Bettvolumen Methanol eluiert. Das Eluat wird im Vakuum zur Trockne eingeengt. Ein solches Eluat oder ein solcher Rohextrakt kann mit geeigneten Dispersions- und/oder Streckmitteln zu gewerblich verwendbaren Pflanzenschutzmitteln formuliert werden, der Rohextrakt kann aber, auch wie im folgenden und in der beigefügten Figur 2 dargestellt, zur Isolierung der einzelnen Soraphene A bis σ, aufgetrennt werden. Die hier beschriebene Trennung wurde mit 65 g Rohextrakt aus insgesamt 400 l Fermentationsbrühe durchgeführt.

Chromatographische Aufreinigung (siehe: Trennungsgang II zur Isolierung von Soraphenen)

1 Kieselgeltrennung; Säule: 200 mm ∅; 200 mm Länge;
Sorbens: Lichroprep Si 100; 25-40 µm (Hersteller: Merck)
Laufmittel: Gradient in Stufen; 1. Dichlormethan; 2.-9. Dichlormethan/Aceton 98/2, 96/4, 95/5, 90/10, 85/15, 80/20, 50/50, 25/75; 10.-11. Dichlormethan/Methanol 75/25, 50/50;
Es werden 14 Fraktionen genommen.
Die Fraktionen 2, 3, 5, 6, 7, 8, 9, 10, 11 werden weiter aufgereinigt.

2 Gelchromatographie; Säule: 60 mm ∅; 100 cm Länge;
Sorbens: Sephadex LH-20 (Hersteller: Pharmacia LKB GmbH)
Laufmittel: Methanol
Es werden 7 Fraktionen genommen.
Die Fraktion 3 enthält Soraphen A (2,5 g).

3 Gelchromatographie; Säule: 30 mm ∅; 100 cm Länge;
Sorbens: Sephadex LH-20 (Hersteller: Pharmacia LKB GmbH)
Laufmittel: Methanol

Es werden 5 Fraktionen genommen.
Die Fraktion 2 enthält Soraphen C (6,6 g).

4 Gelchromatographie; Säule: 30 mm Ø; 100 cm Länge;
Sorbens: Sephadex LH-20 (Hersteller: Pharmacia LKB GmbH)
Laufmittel: Methanol
Es werden 6 Fraktionen genommen.
Die Fraktion 2 (1 g) wird weiter aufgereingt.

5 Reversed-Phase-Trennung; Säule: 37 mm Ø; 34 cm Länge;
Sorbens: HDSIL RP-18; 18-20-60 15-25 µm (Hersteller: Labomatic)
Laufmittel: Methanol/Wasser 72/28.
Es werden 9 Fraktionen genommen.
Die Fraktion 7 enthält Soraphen V (1,1 g).

6 Gelchromatographie; Säule: 30 mm Ø; 100 cm Länge;
Sorbens: Sephadex LH-20 (Hersteller: Pharmacia LKB GmbH)
Laufmittel: Methanol
Es werden 4 Fraktionen genommen.
Die Fraktion 2 (1,1 g) wird weiter aufgereingt.

7 Gelchromatographie; Säule: 30 mm Ø; 100 cm Länge;
Sorbens: Sephadex LH-20 (Hersteller: Pharmacia LKB GmbH)
Laufmittel: Methanol
Es werden 6 Fraktionen genommen.
Die Fraktionen 2 (0,54 g) und 3 (0,18 g) werden weiter aufgereingt.

8 Gelchromatographie; Säule: 30 mm Ø; 100 cm Länge;
Sorbens: Sephadex LH-20 (Hersteller: Pharmacia LKB GmbH)
Laufmittel: Methanol
Es werden 6 Fraktionen genommen.
Die Fraktionen 2 (0,3 g) und 3 (1,3 g) werden weiter aufgereingt.

9 Gelchromatographie; Säule: 60 mm Ø; 100 cm Länge;
Sorbens: Sephadex LH-20 (Hersteller: Pharmacia LKB GmbH)
Laufmittel: Methanol
Es werden 7 Fraktionen genommen.
Die Fraktion 2 (3,3 g) wird weiter aufgereingt.

10 Gelchromatographie; Säule: 60 mm Ø; 100 cm Länge;
Sorbens: Sephadex LH-20 (Hersteller: Pharmacia LKB GmbH)
Laufmittel: Methanol
Es werden 6 Fraktionen genommen.
Die Fraktion 2 (6,1 g) wird weiter aufgereingt.

11 Reversed-Phase-Trennung; Säule: 37 mm Ø; 34 cm Länge;
Sorbens: HDSIL RP-18; 18-20-60 15-25 µm (Hersteller: Labomatic)
Laufmittel: Methanol/Wasser 75/25
Es werden 8 Fraktionen genommen.
Die Fraktion 5 enthält Soraphen D (40 mg).
Die Fraktion 7 enthält Soraphen B (0,5 g).

12 Reversed-Phase-Trennung; Säule: 35 mm Ø; 45 cm Länge;
Sorbens: HDSIL RP-18; 18-30-60 25-40 µm (Hersteller: Labomatic)
Laufmittel: Methanol/Wasser 80/20
Es werden 10 Fraktionen genommen.
Die Fraktion 8 enthält Soraphen E (30 mg).
Die Fraktion 4 wird weiter aufgereinigt.

13 Reversed-Phase-Trennung; Säule: 37 mm Ø; 34 cm Länge;
Sorbens: HDSIL RP-18; 18-20-60 15-25 µm (Hersteller: Labomatic)
Laufmittel: Methanol/Wasser 75/25
Es werden 10 Fraktionen genommen.
Die Fraktion 6 (80 mg) wird weiter aufgereinigt.
Die Fraktion 8 (20 mg) wird weiter aufgereinigt.

14 Reversed-Phase-Trennung; Säule: 37 mm Ø; 34 cm Länge;
Sorbens: HDSIL RP-18; 18-20-60 15-25 µm (Hersteller: Labomatic)
Laufmittel: Methanol/Wasser 75/25
Es werden 11 Fraktionen genommen.
Die Fraktion 5 enthält Soraphen X (16 mg).

15 Reversed-Phase-Trennung; Säule: 35 mm Ø; 45 cm Länge;
Sorbens: HDSIL RP-18; 18-30-60 25-40 µm (Hersteller: Labomatic)
Laufmittel: Methanol/Wasser 70/30 lin. Gradient in 2 h nach Methanol.
Es werden 8 Fraktionen genommen.
Die Fraktion 5 enthält Soraphen U (150 mg).

16 Reversed-Phase-Trennung; Säule: 35 mm ∅; 45 cm Länge;
Sorbens: HDSIL RP-18; 18-30-60 25-40 µm (Hersteller: Labomatic)
Laufmittel: Methanol/Wasser 75/25 lin. Gradient in 1 h nach 90/10.
Es werden 9 Fraktionen genommen.
Die Fraktion 8 enthält Soraphen M (0,93 g).
Die Fraktion 7 (250 mg) wird weiter aufgereinigt.

17 Reversed-Phase-Trennung; Säule: 37 mm ∅; 34 cm Länge;
Sorbens: HDSIL RP-18; 18-20-60 15-25 µm (Hersteller: Labomatic)
Laufmittel: Methanol/Wasser 70/30.
Es werden 13 Fraktionen genommen.
Die Fraktion 4 (105 mg) wird weiter aufgereinigt.
Die Fraktion 6 enthält Soraphen R (20 mg).
Die Fraktion 7 enthält Soraphen S (490 mg).
Die Fraktion 8 enthält Soraphen T (80 mg).
Die Fraktion 9 (80 mg) wird weiter aufgereinigt.

18 Reversed-Phase-Trennung; Säule: 37 mm ∅; 34 cm Länge;
Sorbens: HDSIL RP-18; 18-20-60 15-25 µm (Hersteller: Labomatic)
Laufmittel: Methanol/Wasser 70/30.
Es werden 14 Fraktionen genommen.
Die Fraktion 7 (700 mg) wird weiter aufgereinigt.
Die Fraktion 8 (570 mg) wird weiter aufgereinigt.
Die Fraktion 9 enthält Soraphen δ (120 mg).

19 Reversed-Phase-Trennung; Säule: 20,5 mm ∅; 25 cm Länge;
Sorbens: RP-18 Nucleosil 100-7 $C_{18}$; 7 µm (Hersteller: Macherey Nagel)
Laufmittel: Methanol/Wasser 67/33.
Es werden 6 Fraktionen genommen.
Die Fraktion 5 enthält Soraphen γ (11 mg).

20 Kieselgel-Trennung; Säule: 20,5 mm ∅; 25 cm Länge;
Sorbens: Si 100 Nucleosil 100-7; 7 µm (Hersteller: Macherey Nagel)
Laufmittel: tert. Butylmethylether/n-Hexan/+ Methanol 1/2/+ 1 %
Es werden 3 Fraktionen genommen.
Die Fraktion 2 enthält Soraphen ν (8 mg).

21 Kieselgel-Trennung; Säule: 20,5 mm ∅; 25 cm Länge;
Sorbens: Si 100 Nucleosil 100-7; 7 µm (Hersteller: Macherey Nagel)
Laufmittel: tert. Butylmethylether/n-Hexan/+ Methanol 1/2/+ 1 %
Es werden 2 Fraktionen genommen.
Die Fraktion 2 enthält Soraphen β (8 mg).

22 Kieselgel-Trennung; Säule: 20,5 mm ∅; 25 cm Länge;
Sorbens: Si 100 Nucleosil 100-7; 7 µm (Hersteller: Macherey Nagel)
Laufmittel: tert. Butylmethylether/n-Hexan/+ Methanol 1/2/+ 5 %
Es werden 7 Fraktionen genommen.
Die Fraktion 1 enthält Soraphen Y (64 mg).
Die Fraktion 4 enthält Soraphen ο (5 mg).
Die Fraktion 6 enthält Soraphen ξ (7 mg).

23 Reversed-Phase-Trennung; Säule: 20,5 mm ∅; 25 cm Länge;
Sorbens: RP-18 Nucleosil 100-7 $C_{18}$; 7 µm (Hersteller: Macherey Nagel)
Laufmittel: Methanol/Wasser 60/40.
Es werden 6 Fraktionen genommen.
Die Fraktion 2 (35 mg) wird weiter aufgereinigt.

24 Kieselgel-Trennung; Säule: 20,5 mm ∅; 25 cm Länge;
Sorbens: Si 100 Nucleosil 100-7; 7 µm (Hersteller: Macherey Nagel)
Laufmittel: tert. Butylmethylether/n-Hexan/+ Methanol 1/2/+ 10 %
Es werden 5 Fraktionen genommen.
Die Fraktion 2 enthält Soraphen σ (20 mg).

25 Reversed-Phase-Trennung; Säule: 20.5 mm ∅; 25 cm Länge;
Sorbens: RP-18 Nucleosil 100-7 $C_{18}$; 7 µm (Hersteller: Macherey Nagel)
Laufmittel: Methanol/Wasser 60/40.
Es werden 9 Fraktionen genommen.
Die Fraktion 4 (194 mg) wird weiter aufgereinigt.
Die Fraktion 5 (130 mg) wird weiter aufgereinigt.
Die Fraktion 6 (50 mg) wird weiter aufgereinigt.

26 Kieselgel-Trennung; Säule: 20,5 mm ∅; 25 cm Länge;
Sorbens: Si 100 Nucleosil 100-7; 7 µm (Hersteller: Macherey Nagel)
Laufmittel: tert. Butylmethylether/n-Hexan/+ Methanol 1/2/+ 5 %
Es werden 9 Fraktionen genommen.

Die Fraktion 4 enthält Soraphen ρ (45 mg).

27 Kieselgel-Trennung; Säule: 20,5 mm Ø; 25 cm Länge;
Sorbens: Si 100 Nucleosil 100-7; 7 μm (Hersteller: Macherey Nagel)
Laufmittel: tert. Butylmethylether/n-Hexan/ + Methanol 1/2/ + 10 %
Es werden 4 Fraktionen genommen.
Die Fraktion 2 enthält Soraphen ζ (26 mg).

28 Kieselgel-Trennung; Säule: 20,5 mm Ø; 25 cm Länge;
Sorbens: Si 100 Nucleosil 100-7; 7 μm (Hersteller: Macherey Nagel)
Laufmittel: tert. Butylmethylether/n-Hexan/ + Methanol 1/2/ + 5 %
Es werden 4 Fraktionen genommen.
Die Fraktion 2 enthält Soraphen μ (18 mg).
Die Fraktion 3 enthält Soraphen η (69 mg).

29 Kieselgel-Trennung; Säule: 20,5 mm Ø; 25 cm Länge;
Sorbens: Si 100 Nucleosil 100-7; 7 μm (Hersteller: Macherey Nagel)
Laufmittel: tert. Butylmethylether/n-Hexan/ + Methanol 1/2/ + 5 %
Es werden 5 Fraktionen genommen.
Die Fraktion 4 enthält Soraphen κ (5,5 mg).

30 Kieselgel-Trennung; Säule: 20,5 mm Ø; 25 cm Länge;
Sorbens: Si 100 Nucleosil 100-7; 7 μm (Hersteller: Macherey Nagel)
Laufmittel: tert. Butylmethylether/n-Hexan/ + Methanol 1/2/ + 5 %
Es werden 4 Fraktionen genommen.
Die Fraktion 4 (10 mg) wird weiter aufgereinigt.

31 Kieselgel-Trennung; Säule: 20,5 mm Ø; 25 cm Länge;
Sorbens: Si 100 Nucleosil 100-7; 7 μm (Hersteller: Macherey Nagel)
Laufmittel: tert. Butylmethylether/n-Hexan/ + Methanol 1/2/ + 5 %
Es werden 2 Fraktionen genommen.
Die Fraktion 1 enthält Soraphen π (2,2 mg).
Die Fraktion 2 enthält Soraphen Z (6,5 mg).

Physikochemische Charakterisierung der weiterhin gewonnenen Soraphene R bis σ

Soraphen V
$C_{28}H_{42}O_8$
Mz 506
IR (Film, ν in $cm^{-1}$)
3394; 2942; 2900; 2829; 1723; 1461; 1380; 1270; 1233; 1189; 1068; 988; 898; 851.
$^{13}C$-NMR-Verschiebungen ($CDCl_3$; δ in ppm)
10.3; 11.7; 12.6; 23.2; 26.0; 32.7; 35.3; 35.6; 36.2; 46.3; 55.8; 57.3; 68.9; 72.5; 72.9; 74.6; 76.3; 84.5; 99.5; 122.1; 126.2; 126.2; 128.1; 128.5; 128.5; 139.9; 141.2; 170.9.
HPLC $R_t$ = 9,2 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen X
$C_{28}H_{42}O_9$
Mz 522
IR (Film, ν in $cm^{-1}$)
3404; 2941; 1716; 1598; 1461; 1380; 1272; 1233; 1189; 1156; 1100; 1069; 988.
$^{13}C$-NMR-Verschiebungen ($CDCl_3$; δ in ppm)
10.3; 11.7; 12.5; 23.1; 25.9; 29.4; 35.2; 35.6; 35.7; 46.2; 57.3; 68.9; 72.5; 74.5; 75.0; 76.1; 83.8; 99.5; 113.4; 115.2; 118.0; 124.9; 129.8; 137.4; 142.7; 156.2; 170.9.
HPLC $R_t$ = 4,4 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen U
$C_{29}H_{44}O_9$
Mz 536
IR (Film, ν in $cm^{-1}$)
3386; 3361; 2977; 2939; 2892; 2823; 1698; 1461; 1380; 1268; 1185; 1152; 1096; 1064; 1023; 975; 900; 840.
$^{13}C$-NMR-Verschiebungen ($CDCl_3$; δ in ppm)
10.1; 11.4; 12.2; 31.8; 32.9; 35.2; 35.2; 38.6; 46.3; 56.0; 57.2; 57.9; 64.2; 68.7; 72.2; 73.3; 76.2; 80.0; 84.5; 99.3;

122.2; 126.1; 126.1; 127.9; 128.4; 128.4; 140.6; 141.4; 171.7.
HPLC $R_t$ = 5,2 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen R
$C_{26}H_{40}O_8$
Mz 480
IR (Film, ν in $cm^{-1}$)
3404; 2937; 1731; 1459; 1430; 1384; 1355; 1330; 1270; 1212; 1110; 1083; 1033; 988.
$^{13}$C-NMR-Verschiebungen ($CDCl_3$; δ in ppm)
10.7; 14.5; 22.7; 23.3; 24.8; 27.9; 30.9; 31.8; 35.5; 35.3; 43.4; 58.6; 68.9; 71.0; 72.9; 73.3; 74.6; 80.5; 97.5; 126.5; 126.5; 128.0; 128.6; 128.6; 141.1; 168.8.
HPLC $R_t$ = 5,1 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen S
$C_{27}H_{40}O_8$
Mz 492
IR (Film, ν in $cm^{-1}$)
3431; 2941; 1720; 1604; 1459; 1426; 1380; 1264; 1187; 1150; 1104; 1062; 977.
$^{13}$C-NMR-Verschiebungen ($CDCl_3$; δ in ppm)
10.5; 11.9; 12.7; 24.9; 26.2; 35.5; 36.2; 36.3; 37.9; 47.5; 57.6; 69.5; 73.5; 74.1; 75.8; 76.3; 77.9; 100.6; 125.1; 127.1; 127.1; 128.7; 129.4; 129.4; 138.8; 143.3; 173.8.
HPLC $R_t$ = 5,9 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen T
$C_{27}H_{42}O_8$
Mz 494
IR (Film, ν in $cm^{-1}$)
3427; 2941; 1720; 1461; 1382; 1268; 1191; 1154; 1106; 1068; 994; 971.
$^{13}$C-NMR-Verschiebungen ($CDCl_3$; δ in ppm)
10.4; 11.7; 14.2; 22.7; 24.8; 25.3; 28.3; 30.6; 32.8; 34.6; 35.2; 45.8; 57.3; 69.1; 69.8; 73.0; 73.3; 76.0; 76.4; 99.6; 126.5; 126.5; 128.1; 128.5; 128.5; 140.6; 171.9.
HPLC $R_t$ = 6,5 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen δ (Delta)
$C_{27}H_{42}O_8$
Mz 506
IR (Film, ν in $cm^{-1}$)
3440; 3311; 2937; 1731; 1600; 1461; 1380; 1340; 1185; 1096; 988; 850.
$^{13}$C-NMR-Verschiebungen ($CDCl_3$; δ in ppm)
10.2; 12.0; 12.7; 23.2; 25.6; 29.6; 34.9; 36.0; 37.3; 45.3; 56.2; 57.6; 70.3; 72.1; 72.6; 74.3; 74.9; 84.2; 99.5; 124.9; 126.3; 126.3; 128.0; 128.6; 128.6; 131.6; 141.2; 171.2.
HPLC $R_t$ = 7,1 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen γ (Gamma)
$C_{28}H_{42}O_9$
Mz 522
IR (Film, ν in $cm^{-1}$)
3398; 2939; 1723; 1461; 1382; 1264; 1189; 1152; 1102; 1066; 975; 894.
$^{13}$C-NMR-Verschiebungen ($CDCl_3$; δ in ppm)
8.6; 10.3; 11.5; 23.6; 25.8; 28.8; 33.9; 35.4; 36.3; 46.6; 54.7; 57.3; 58.1; 59.2; 68.8; 69.3; 73.5; 74.3; 76.2; 83.5;

99.7; 126.1; 126.1; 128.0; 128.6; 128.6; 141.4; 170.4.
HPLC $R_t$ = 8,4 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen ν (Nü)
$C_{28}H_{42}O_9$
Mz 522
IR (Film, ν in $cm^{-1}$)
3402; 2971; 2935; 2827; 1733; 1459; 1367; 1181; 1095; 1050; 990; 860.
$^{13}$C-NMR-Verschiebungen ($CDCl_3$; δ in ppm)
8.7; 10.3; 11.5; 26.3; 33.4; 35.2; 40.2; 40.8; 46.2; 57.4; 58.1; 67.8; 68.9; 71.5; 73.5; 76.3; 78.9; 82.6; 99.4; 125.9; 125.9; 128.3; 128.7; 128.7; 128.8; 129.5; 140.7; 170.3.
HPLC $R_t$ = 8,5 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen β (Beta)
$C_{29}H_{44}O_8$
Mz 520
IR (Film, ν in $cm^{-1}$)
3408; 2971; 2935; 2825; 1733; 1459; 1365; 1343; 1181; 1095; 1050; 990; 958; 842; 738.
$^{13}$C-NMR-Verschiebungen ($CDCl_3$; δ in ppm)
10.1; 12.1; 12.6; 23.4; 25.5; 30.4; 35.0; 35.9; 37.2; 45.3; 56.2; 56.3; 57.9; 70.3; 72.1; 72.7; 74.2; 83.1; 84.8; 99.4; 122.6; 126.3; 126.3; 128.0; 128.6; 128.6; 140.0; 141.3; 171.3.
HPLC $R_t$ = 16,8 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen Y
$C_{27}H_{40}O_9$
Mz 508
IR (Film, ν in $cm^{-1}$)
3436; 2937; 1721; 1459; 1376; 1268; 1199; 1114; 1048; 988; 961.
$^{13}$C-NMR-Verschiebungen ($CDCl_3$; δ in ppm)
10.8; 11.2; 18.8; 23.0; 23.5; 31.7; 33.4; 36.2; 40.6; 41.1; 51.1; 61.1; 70.8; 74.6; 76.4; 77.1; 78.7; 86.4; 99.9; 125.5; 125.5; 127.4; 128.4; 128.4; 142.5; 171.1; 212.4.
HPLC $R_t$ = 6,1 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen o (Omicron)
$C_{29}H_{44}O_9$
Mz 536
IR (Film, ν in $cm^{-1}$)
3402; 3361; 2933; 2894; 1708; 1457; 1360; 1269; 1187; 1150; 1096; 1062; 975; 898; 838; 765.
$^{13}$C-NMR-Verschiebungen ($CDCl_3$; δ in ppm)
10.4; 11.6; 12.6; 31.7; 33.4; 35.3; 35.7; 38.7; 46.3; 56.3; 57.4; 58.3; 65.7; 69.0; 72.3; 74.2; 76.2; 80.0; 84.8; 99.5; 122.9; 126.3; 126.3; 128.2; 128.6; 128.6; 140.1; 140.7; 170.8.
HPLC $R_t$ = 4,8 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen ξ (Xi)
$C_{27}H_{40}O_8$
Mz 492
IR (Film, ν in $cm^{-1}$)
3402; 2969; 2933; 1731; 1459; 1367; 1179; 1095; 1050; 990.
$^{13}$C-NMR-Verschiebungen ($CDCl_3$; δ in ppm)
10.4; 11.7; 12.5; 20.1; 26.2; 31.8; 35.2; 35.7; 36.2; 46.4; 57.4; 68.9; 72.3; 73.5; 75.0; 76.1; 76.3; 99.6; 126.2;

126.2; 127.0; 128.1; 128.6; 128.6; 138.3; 141.2; 170.7.
HPLC $R_t$ = 4,1 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen $\sigma$ (Sigma)
$C_{29}H_{44}O_9$
Mz 536
IR (Film, $\nu$ in $cm^{-1}$)
3398; 3363; 3301; 2971; 2933; 2894; 1702; 1453; 1378; 1262; 1167; 1150; 1100; 979; 838.
$^{13}C$-NMR-Verschiebungen ($CDCl_3$; $\delta$ in ppm)
10.3; 11.5; 12.4; 31.6; 34.2; 35.0; 35.5; 38.9; 46.2; 56.1; 57.5; 58.5; 64.3; 69.1; 73.1; 76.1; 77.2; 79.8; 85.2; 99.7; 121.8; 126.0; 126.0; 128.0; 128.5; 128.5; 141.9; 142.0; 171.4.
HPLC $R_t$ = 6,2 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen $\rho$ (Rho)
$C_{27}H_{42}O_9$
Mz 510
IR (Film, $\nu$ in $cm^{-1}$)
3398; 3363; 3301; 2971; 2933; 2894; 1700; 1453; 1378; 1262; 1187; 1150; 1100; 1064; 979; 898; 838; 765.
$^{13}C$-NMR-Verschiebungen ($CDCl_3$; $\delta$ in ppm)
8.7; 10.3; 11.4; 23.1; 25.5; 26.4; 32.3; 35.0; 36.1; 40.5; 46.6; 57.2; 68.5; 68.8; 71.8; 73.1; 73.7; 74.3; 76.4; 76.4; 99.7; 126.3; 127.9; 128.5; 128.5; 141.4; 171.4.
HPLC $R_t$ = 4,7 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen $\zeta$ (Zeta)
$C_{28}H_{42}O_9$
Mz 522
IR (Film, $\nu$ in $cm^{-1}$)
3398; 2937; 1718; 1457; 1378; 1332; 1272; 1203; 1102; 978.
$^{13}C$-NMR-Verschiebungen ($CDCl_3$; $\delta$ in ppm)
11.8; 21.9; 25.9; 25.9; 28.7; 36.3; 39.9; 43.0; 45.7; 56.7; 57.4; 58.6; 70.6; 75.5; 76.2; 78.2; 78.2; 84.3; 98.9; 125.9; 125.9; 128.0; 128.4; 128.7; 128.7; 133.1; 141.2; 171.6.
HPLC $R_t$ = 3,5 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen $\mu$ (Mü)
$C_{28}H_{44}O_9$
Mz 524
IR (Film, $\nu$ in $cm^{-1}$)
3423; 2939; 1725; 1459; 1380; 1264; 1191; 1154; 1102; 1052; 992; 971.
$^{13}C$-NMR-Verschiebungen ($CDCl_3$; $\delta$ in ppm)
10.4; 11.7; 14.1; 21.3; 27.7; 28.2; 30.9; 32.9; 34.1; 35.2; 45.6; 57.4; 60.7; 69.1; 70.0; 70.4; 72.1; 76.2; 76.3; 85.4; 99.6; 126.6; 126.6; 128.2; 128.6; 128.6; 140.2; 172.0.
HPLC $R_t$ = 4,7 min
Säule: 4 x 250 mm Nucleosil 100-7 $C_{18}$, Macherey Nagel:
Fluss: 1,5 ml/min; Laufmittel: Methanol/Wasser 70/30;
Detektor: UV 210 nm.

Soraphen $\eta$ (Eta)
$C_{28}H_{42}O_9$
Mz 522
IR (Film, $\nu$ in $cm^{-1}$)
3411; 2935; 1718; 1459; 1382; 1266; 1189; 1152; 1104; 1066; 981.
$^{13}C$-NMR-Verschiebungen ($CDCl_3$; $\delta$ in ppm)
10.2; 11.4; 12.3; 31.6; 32.9; 35.1; 35.4; 38.1; 46.2; 57.2; 57.7; 64.0; 68.8; 72.2; 73.7; 74.7; 76.2; 81.2; 99.3; 124.8;

126.1; 126.1; 127.9; 128.4; 128.4; 137.8; 141.1; 171.4.
HPLC $R_t$ = 4,6 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen κ (Kappa)
$C_{27}H_{40}O_8$
Mz 492
IR (Film, ν in $cm^{-1}$)
3411; 2935; 1731; 1461; 1382; 1347; 1270; 1189; 1104; 1048; 990.
$^{13}C$-NMR-Verschiebungen ($CDCl_3$; δ in ppm)
10.1; 12.1; 12.8; 23.4; 25.4; 33.5; 35.0; 36.1; 37.2; 45.4; 56.1; 70.3; 72.0; 72.7; 74.2; 74.2; 75.6; 99.4; 124.1; 126.2; 126.2; 127.9; 128.5; 128.5; 138.6; 141.3; 171.5.
HPLC $r_t$ = 4,7 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen π (Pi)
$C_{27}H_{40}O_8$
Mz 492
IR (Film, ν in $cm^{-1}$)
3396; 3363; 3299; 2969; 2933; 2694; 1702; 1451; 1378; 1262; 1167; 1150; 1100; 979.
$^{13}C$-NMR-Verschiebungen ($CDCl_3$; δ in ppm)
10.3; 12.3; 14.6; 23.4; 25.0; 32.2; 35.6; 36.0; 36.8; 50.4; 55.9; 70.2; 71.2; 73.9; 75.0; 75.5; 98.3; 125.2; 126.2; 126.2; 128.1; 128.6; 128.6; 137.8; 141.0; 171.5.
HPLC $R_t$ = 4,9 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Soraphen Z
$C_{28}H_{42}O_9$
Mz 522
IR (Film, ν in $cm^{-1}$)
3405; 2937; 1718; 1459; 1380; 1266; 1189; 1152; 1104; 1069; 987; 902; 850; 813; 738.
$^{13}C$-NMR-Verschiebungen ($CDCl_3$; δ in ppm)
10.5; 11.7; 12.6; 31.8; 33.5; 35.3; 35.7; 41.1; 46.3; 56.0; 57.3; 66.0; 69.0; 70.5; 72.5; 74.3; 76.2; 84.3; 99.5; 122.4; 126.2; 126.2; 128.2; 128.6; 128.6; 140.4; 140.9; 170.9.
HPLC $R_t$ = 7,7 min
Säule: 4 x 250 mm Nucleosil 100-7 $C_{18}$, Macherey Nagel;
Fluss: 1,5 ml/min; Laufmittel: Methanol/Wasser 70/30;
Detektor: UV 210 nm.

Aufgrund der Röntgenstrukturanalyse des Soraphens A und der vorstehend ermittelten Daten werden für die Soraphene C bis Q die folgenden räumlichen Strukturen IC′ bis IQ′ angenommen:

(IC')

(ID')

(IE')

(IF')

OCH3 21 CH3 OH OH 20 CH3 O O OH O OH OCH3 CH3

(IH')

OCH3 CH3 OCH3 CH3 O O OH O OH OH CH3

(IJ')

OH OH 21 CH3 OCH3 23 20 CH3 O O OH O OH OCH3 CH3

(IM')

OH 21 CH3 OCH3 OCH3 23 20 CH3 O O OH O OH OCH3 CH3

(IN')

(IQ')

Für die restlichen IR', IS', IT', IU', IV', IX', IY', IZ', Iβ', Iγ', Iδ', Iζ', Iη', Iκ', Iμ', Iξ' und Iρ' werden ähnliche Konfigurationen angenommen.

Für Soraphen Iν' ist die folgende Konfiguration wahrscheinlich:

(Iν')

Für die Soraphene ο, π und σ ist die Konfiguration nicht gesichert. Ueberdies ist ersichtlicht, dass einzelne Soraphene untereinander epimer sind. Soraphen δ ist epimer zu Soraphen C; die Sorphene κ und ξ sind untereinander und zu Soraphen S epimer, desgleichen die Soraphene U, ο und σ. Verbindlich sind in jedem Falle die für eine bestimmte Verbindung der Formel I ermittelten physikalischen Messwerte, die, wie der Fachmann weiss, zuweilen keine strenge Zuordnung zu einer bestimmten Konfiguration erlauben.

Wie erwähnt, umfasst die Erfindung sämtliche stereoisomeren Formen der einzelnen aufgeführten Verbindungen der Formel I sowie ihre Herstellung und ihre Verwendung zur Bekämpfung bzw. Verhütung Pflanzenkrankheiten. Des weiteren schliesst die Erfindung aber insbesondere auch die Verbindungen IC' bid Iσ' aufgrund ihrer physikochemischen Daten ein, für die die oben wiedergegebenen Konfigurationon angenommen werden.

Es ist zu beachten, dass die macrocyclischen Soraphene der Formel I normalerweise in der angegebenen Hemiacetalform vorliegen, diese Form jedoch eine reversible Ringöffnung nach dem Schema

erfahren kann. Je nach Herstellungs- bzw. nach Aufarbeitungsmethodik fallen, abhängig vom pH-Wert und vom Lösungsmittel, die Soraphenein der einen oder anderen Form oder als Gemisch beider Formen an. Charakteristisch für die Ringöffnung ist die Verschiebung des $^{13}$C-NMR-Signals in der 3-Position und die der

$^1$H-NMR-Signale in bestimmten anderen Positionen. Beim Soraphen A werden beispielsweise folgende Veränderungen beobachtet: $^{13}$C-NMR(CDCl$_3$, δ in ppm) 99,5 →203,1(3-C).$^1$H-NMR(CDCl$_3$, δ in ppm): 3,14→3,72(2-H); 3,18→4,5(4-H); 3,83→3,16 (7-H); 5,86→5,7 (17-H). Aehnliche Verschiebungen werden auch bei den Soraphenen B bis ρ beobachtet. Die Formel I vorliegender Erfindung umfasst grundsätzlich sowohl die bei niedrigen pH-Werten bevorzugte 3-Hemiacetalform wie auch die geöffnete 3-Keto-7-hydroxyform.

2. Formulierungsbeispiele für einen oder mehrere der Wirkstoffe der Formel I (% = Gewichtsprozent)

Wirkstoff bedeutet im folgenden eines der Soraphene in reiner Form oder mehrere der Soraphene C bis σ der ein aus der mikrobiologischen Herstellung gewonnenes Eluat in flüssiger Form oder als getrockneter Rückstand, worin die unaufgetrennte Gesamtmenge der Soraphene enthalten ist.

| 2.1 Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Stäubemittel

| | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel. Diese lassen sich durch weiteren Zusatz der drei Trägerstoffe auf anwendungsfertige Stäube mit 0,001 % Wirkstoff vermahlen.

| 2.5 Spritzpulver | a) | b) | c) |
|---|---|---|---|
| Wirkstoff | 25 % | 50 % | 75 % |
| Na-Ligninsulfonat | 5 % | 5 % | - % |
| Na-Laurylsulfat | 3 % | - | 5 % |
| Na-Diisobutylnaph thalinsulfonat | - | 6 % | 10 % |
| Octylphenolpolyethy-lenglykolether (7-8 Mol Ethylenoxid) | - | 2 % | - % |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Umhüllungs-Granulat

| | |
|---|---|
| Wirkstoff | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.7 Suspensions-Konzentrat

| | |
|---|---|
| Wirkstoff | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

2.8 Biomasse-Konzentrat

Die aus der Produktionskultur anfallende Biomasse wird getrocknet, vermahlen und im Gewichtsverhältnis 80:20 mit Ethylenglykol-monomethylether gemischt. Ein solches Konzentrat lässt sich beliebig mit Wasser zu Sprühsuspensionen verdünnen.

3. Biologische Beispiele an Pflanzen

(Im folgenden bedeutet "Wirkstoff" einen der Soraphene C bis σ gemäss vorliegender Erfindung)

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspenion des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,002 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Der Pilzbefall wurde in beiden Versuchen vollständig durch den Wirkstoff gehemmt.

Unbehandelte aber infizierte Kontrollpflanzen wiesen dagegen einen Puccinia-Befall von 100 % auf.

Beispiel 3.2: Wirkung gegen Phytophthora auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luchtfeuchtigkeit und 20° C.

b) Systemische Wirkung

Zu Tomatenpflanzen wurde nach 3-wöchiger Anzucht eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangien suspension des Pilzes infiziert. Die Beurteilung des Pilzbefalls erfolgte nach einer Inkubation der infizierten Pflanzen während 5 Tagen bei 90-100 % relativer Luftfeuchtigkeit und 20° C.

In beiden Versuchen wurde bei der Auswertung kein Pilzbefall beobachtet, während die infizierten Kontrollpflanzen vollständig befallen waren.

Beispiel 3.3: Wirkung gegen Plasmopara viticola auf Reben

Residual-protektive Wirkung

Im 4-5 Blattstadium werden Rebensämlinge mit einer aus Spritzpulver des Wirkstoffes hergestellten Spritzbrühe (0,006 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20° C wird der Pilzbefall beurteilt.

Im Gegensatz zu den unbehandelten, aber infizierten Kontrollpflanzen mit 100 % Pilzbefall waren die mit dem Wirkstoff I behandelten Pflanzen befallsfrei.

Beispiel 3.4: Wirkung gegen Cercospora arachidicola auf Erdnusspflanzen

Residual protektive Wirkung

10-15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Die mit dem Wirkstoff I behandelten Pflanzen waren ohne Befall. Die mit Soraphen A behandelten Pflanzen zeigten auch bei Anwendung einer Spritzkonzentration von 0,002 % am Schluss der Auswertung keinen Befall. Unbehandelte aber infizierte Kontrollpflanzen wiesen dagegen einen Cercospora-Befall von 100 % auf.

Beispiel 3.5: Wirkung gegen Venturia inaequalis auf Apfeltrieben

Residual-protektive Wirkung

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24° C aufgestellt. Der Schorfbefall wird 15 Tage nach der Infektion beurteilt.

Die mit dem Wirkstoff behandelten Stecklinge waren befallsfrei, Kontrollpflanzen dagegen vollständig befallen.

Beispiel 3.6: Wirkung gegen Botrytis cinerea an Apfelfrüchten

Residual-protektive Wirkung

Künstlich verletzte Aepfel werden behandelt, indem eine aus Spritzpulver der Wirksubstanz hergestellte Spritzbrühe (0,02 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20° C inkubiert. Bei der Auswertung werden die angefaulten Verletzungsstellen gezählt und daraus die fungizide Wirkung der Testsubstanz abgeleitet.

Der Wirkstoff hemmte den Pilzwuchs vollständig. Unbehandelte aber infizierte Kontrollpflanzen wiesen dagegen 100 % Botrytis-Befall auf.

Beispiel 3.7: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

Die Pflanzen waren in beiden Versuchen befallsfrei, die Kontrollpflanzen vollständig befallen.

Beispiel 3.8: Wirkung gegen Rhizoctonia solani (Bodenpilz auf Reispflanzen)

Protektiv-lokale Bodenapplikation

12 Tage alte Reispflanzen werden mit einer aus einer Formulierung des Wirkstoffes hergestellten Spritzbrühe (0,02 % Aktivsubstanz), ohne oberiidische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27° C (Tag), bzw. 23° C (Nacht) und 100 % rel. Luftfeuchtigkeit (Feuchtkasten) in der Klimakammer wird der Pilzbefall auf Blattscheide, Blättern und Stengel beurteilt.

Nach Behandlung mit dem Wirkstoff trat kein Befall auf, während die infizierten Kontrollpflanzen vollen Rhizoctonia-Befall zeigten.

**Patentansprüche**

1. Verfahren zur Herstellung von Verbindungen der Formel I

(I)

worin der macrocyclische Ring entweder gesättigt oder wahlweise in der 8,9-Stellung oder in der 9,10-Stellung oder in der 14,15-Stellung eine Doppelbindung gemäss nachfolgender Substituentenkombination enthält und die Substituenten $R_1$ bis $R_5$, A, B, X und Y in folgenden kombinierten Bedeutungen vorliegen:

| Verb. | $R_1$ | $R_2$ | $R_3$ | $R_4$ | $R_5$ | A | B | X | Y | Doppelbindung |
|---|---|---|---|---|---|---|---|---|---|---|
| A | $OCH_3$ | $CH_3$ | $CH_3$ | H | H | $CH_3$ | H | H | H | Δ9,10 |
| B | OH | $CH_3$ | $CH_3$ | H | H | $CH_3$ | H | H | H | -- |
| C | OH | $CH_3$ | $CH_3$ | H | H | $CH_3$ | H | H | H | Δ9,10 |
| D | OH | $CH_3$ | $CH_3$ | H | H | H | H | H | H | -- |
| E | $OCH_3$ | $CH_3$ | $CH_3$ | H | H | $CH_3$ | H | OH | H | -- |
| F | $OCH_3$ | $CH_3$ | $CH_3$ | H | H | $CH_3$ | H | H | H | -- |
| H | $OCH_3$ | $CH_3$ | H | H | H | $CH_3$ | H | H | OH | Δ8,9 |
| J | $OCH_3$ | H | $CH_3$ | H | H | $CH_3$ | H | H | H | -- |
| M | OH | $CH_3$ | $CH_3$ | H | H | $CH_3$ | H | OH | H | -- |
| N | OH | $CH_3$ | $CH_3$ | H | H | $CH_3$ | H | H | $OCH_3$ | Δ9,10 |
| Q | $OCH_3$ | $CH_3$ | $CH_3$ | H | H | H | H | H | H | -- |
| R | OH | $CH_3$ | H | H | H | H | H | H | H | -- |
| S | OH | $CH_3$ | H | H | H | $CH_3$ | H | H | H | Δ9,10 |
| T | OH | $CH_3$ | H | H | H | $CH_3$ | H | H | H | -- |
| U | $OCH_3$ | $CH_3$ | $CH_3$ | OH | H | $CH_3$ | H | H | H | Δ9,10 |
| V | $OCH_3$ | $CH_3$ | H | H | H | $CH_3$ | H | H | H | Δ9,10 |
| X | OH | $CH_3$ | H | H | OH | $CH_3$ | H | H | H | Δ9,10 |
| Y | =O | $CH_3$ | H | H | H | $CH_3$ | H | OH | H | -- |
| Z | $OCH_3$ | $CH_3$ | H | OH | H | $CH_3$ | H | H | H | Δ9,10 |
| β | $OCH_3$ | $CH_3$ | $CH_3$ | H | H | $CH_3$ | H | H | H | Δ9,10 |
| γ | OH | $CH_3$ | $CH_3$ | H | H | $CH_3$ | H | 9,10-epoxy | | -- |
| δ | OH | $CH_3$ | $CH_3$ | H | H | $CH_3$ | H | H | H | Δ9,10 |
| ζ | OH | $CH_3$ | $CH_3$ | H | H | $CH_3$ | OH | H | H | Δ9,10 |
| η | OH | $CH_3$ | $CH_3$ | OH | H | $CH_3$ | H | H | H | Δ9,10 |
| κ | OH | $CH_3$ | H | H | H | $CH_3$ | H | H | H | Δ9,10 |
| μ | $OCH_3$ | $CH_3$ | H | H | H | $CH_3$ | H | H | OH | -- |
| ν | OH | $CH_3$ | $CH_3$ | H | H | $CH_3$ | H | OH | H | Δ14,15 |
| ε | OH | $CH_3$ | H | H | H | $CH_3$ | H | H | H | Δ9,10 |
| ο | $OCH_3$ | $CH_3$ | $CH_3$ | OH | H | $CH_3$ | H | H | H | Δ9,10 |
| π | OH | $CH_3$ | H | H | H | $CH_3$ | H | H | H | Δ9,10 |
| ρ | OH | $CH_3$ | H | H | H | $CH_3$ | H | OH | H | -- |
| σ | $OCH_3$ | $CH_3$ | $CH_3$ | OH | H | $CH_3$ | H | H | H | Δ9,10 |

unter Einschluss ihrer Stereoisomeren, gekennzeichnet durch aerobe Züchtung wahlweise eines StammesSorangium cellulosum "So ce 26" (NCIB 12411),
Sorangium cellulosum "So ce 139" (DSM 5397),
Sorangium cellulosum "So ce 170" (DSM 4795),

Sorangium cellulosum "So ce 191" (DSM 4796),
Sorangium cellulosum "So ce 192" (DSM 4797),
Sorangium cellulosum "So ce 231" (DSM 5393) oder
Sorangium cellulosum "So ce 242" (DSM 5414)
oder einer von diesem ableitbaren Kultur in einem geeigneten Nährmedium, und, sofern gewünscht, nachfolgende Abtrennung der erhaltenen Verbindungen der Formel I, mit der Massgabe, dass der Stamm "So ce 26" (NCIB 12411) nicht für die Gewinnung der Verbindungen IA und IB eingesetzt wird.

2. Verfahren gemäss Anspruch 1, dadurch gekennzeichnet, dass man einen einen der genannten sieben Stämme von Sorangium cellulosum in einem Kulturmedium enthaltend mindestens je eine assimilationsfähige C-Quelle und N-Quelle sowie entsprechende anorganische Salze, bei 10-40°C in Anwesenheit oder Abwesenheit eines Adsorberharzes kultiviert, dann die Kulturbrühe bzw. das abfiltrierte Adsorberharz mit einer geeigneten Lösungsmittelphase extrahiert, die erhaltene Lösung einengt und den verbleibenden Rückstand, sofern gewünscht, durch Chromatographie und/oder Umkristallisation reinigt.

3. Verfahren gemäss Anspruch 2, dadurch gekennzeichnet, dass die Fermentation bei 10-35°C mit einem der Stämme
"So ce 170" (DSM 4795)
"So ce 191" (DSM 4796)
"So ce 192" (DSM 4797)
durchgeführt wird.

4. Die nach dem Verfahren gemäss Anspruch 1 erhaltenen Verfahrensprodukte.

5. Die nach dem Verfahren gemäss Anspruch 1 erhaltenen für agrarchemische Zwecke einsetzbaren Kulturmedien enthaltend die Verfahrensprodukte.

6. Agrarchemisch verwendbare Rohextrakte aus dem Kulturmedium des Verfahrens gemäss Anspruch 1.

7. Macrocyclische Verbindungen der Formel I gemäss Anspruch 1 und ihre Stereoisomeren in reiner Form, mit Ausnahme der Verbindungen IA und IB.

8. Die Verbindung IY und die Verbindung Iρ (= Rho) gemäss Anspruch 1 in reiner Form.

9. Mittel zur Bekämpfung bzw. Verhütung von Pflanzenkranheiten, dadurch gekennzeichnet, dass es als Wirkstoff ein Verfahrensprodukt der Formel I des Verfahrens gemäss Anspruch 1 mit Ausnahme der Verbindungen IA und IB enthält.

10. Verwendung der nach dem Verfahren gemäss Anspruch 1 erhaltenen allgemeinen Verfahrensprodukte zur Bekämpfung bzw. Verhütung von Pflanzenkrankheiten, mit Ausnahme der Verbindungen IA und IB.

11. Verwendung gemäss Anspruch 10, dadurch gekennzeichnet, dass die Verfahrensprodukte solche der Formel I oder ihrer Stereoisomeren sind.

12. Mikroorganismus, der befähigt ist, eine Verbindung der Formel I gemäss Anspruch 1 herzustellen, mit Ausnahme des Mikroorganismus Sorangium (Polyangium) cellulosum "So ce 26" (NCIB 12411) oder einer Mutante oder einer Rekombinante von diesem letzteren.

13. Eine biologisch reine Kultur, ausgewählt aus Sorangium cellulosum "So ce 139" (DSM 5397),
Sorangium cellulosum "So ce 170" (DSM 4795),
Sorangium cellulosum "So ce 191" (DSM 4796),
Sorangium cellulosum "So ce 192" (DSM 4797),
Sorangium cellulosum "So ce 231" (DSM 5393)
und Sorangium cellulosum "So ce 242" (DSM 4797),
die befähigt ist, mindestens eine Verbindung der Formel I gemäss Anspruch 1 herzustellen.

14. Eine biologisch ableitbare Kultur eines der Mikroorganismen gemäss Anspruch 13, die zur Produktion einer Verbindung der Formel I befähigt ist.

15. Eine biologisch ableitbare Kultur gemäss Anspruch 13, die zur Produktion von mindestens zwei Verbindungen der Formel I, unter Einschluss der Verbindung A, befähigt ist.

FIGUR 1

Trennungsgang zur Isolierung von Soraphenen

XAD-Eluat

1 Si 100

2 LH 20

3 RP 18

4 Si 100

5 Si 100

6 Si 60

7 RP 18

8 RP 18

9 RP 18

10 RP 18

11 Si 100

12 Si 100

13 Si 100

14 RP 18

15 RP 18

16 RP 18

17 RP 18

A B C D E F H I M N Q

○ Chromatographie 1-17

○ Soraphen A .. Q

FIGUR 2
Trennungsgang zur Isolierung von Soraphenen II
XAD-Eluat
Chromatographie 1 - 31
Soraphen A .. ρ